# EUROPEAN PATENT APPLICATION

(11) **EP 4 286 411 A1**
(43) Date of publication of application: **06.12.2023**
(21) Application number: 22745298.4
(22) Date of filing: 27.01.2022
(51) Int. Cl.: C07K 16/28, C12N 15/13, A61K 39/395, A61P 35/00, A61P 35/04

(54) **HUMANIZED ANTIBODY AGAINST TNFR2 AND USE THEREOF**

(30) Priority: 29.01.2021 CN 202110140980; 31.08.2021 CN 202111016307
(71) Applicant: Shandong Simcere Biopharmaceutical Co., Ltd., Shandong 264006 (CN)
(72) Inventor: ZHAO, Xiaofeng, Nanjing, Jiangsu 210042 (CN); LU, Shiqiang, Nanjing, Jiangsu 210042 (CN); CAO, Zhuoxiao, Nanjing, Jiangsu 210042 (CN); TANG, Renhong, Nanjing, Jiangsu 210042 (CN); REN, Jinsheng, Nanjing, Jiangsu 210042 (CN)
(74) Representative: HGF
(86) International application number: PCT/CN2022/074228
(87) International publication number: WO 2022/161425

(57) **Abstract**

Disclosed in the present invention are a humanized antibody that can specifically bind to TNFR2, or an antigen-binding fragment thereof. The humanized antibody or antigen-binding fragment thereof can regulate the function of immune cells and can be used in a drug for treating diseases related to immune abnormalities, such as tumours.

## Description

The present application claims the right of priority for Chinese patent application No. 202110140980.5, entitled "HUMANIZED ANTIBODY AGAINST TNFR2 AND USE THEREOF" and submitted to the China National Intellectual Property Administration on January 29, 2021, and for Chinese patent application No. 202111016307.7, entitled "HUMANIZED ANTIBODY AGAINST TNFR2 AND USE THEREOF" and submitted to the China National Intellectual Property Administration on August 31, 2021, the contents of which are all incorporated herein by reference in their entireties.

### Technical Field

The present invention relates to the fields of biomedicine and bioengineering, in particular to a humanized antibody against TNFR2 or an antigen-binding fragment thereof, a pharmaceutical composition of the humanized antibody against TNFR2 or antigen-binding fragment thereof and the use thereof.

### Background Art

Immunity is a protective response of the body, which is affected by many genes, proteins and cells. Immune abnormalities may cause many diseases, including tumours, immune deficiencies (e.g., acquired immune deficiency syndrome), allergies, rheumatoid arthritis and other diseases. In the past few years, tumour immunotherapy, as a brand-new treatment method, has become a hot spot in the field of tumour treatment research. Antagonistic antibodies targeting immune checkpoint proteins, such as anti-PD-1 and anti-CTLA-4 monoclonal antibodies, have been used to treat many types of cancers, and have achieved revolutionary results, greatly prolonging the life expectancy of patients with malignant tumours. However, there are still many cancer patients who do not respond to treatment with antagonistic antibodies against immune checkpoint proteins or develop resistance or drug resistance after short-term treatment. Therefore, it is necessary to develop new drugs for treating cancers, which can be used alone or in combination with other tumour treatment methods, including in combination with antagonistic antibodies against immune checkpoint proteins, to further improve the efficacy and safety.

The present inventors have found that human TNFR2 is overexpressed on the surface of human Tregs and various human tumour cells, suggesting that human TNFR2 may promote the tumourigenesis in patients and mediate the immunosuppression and immune escape in the tumour microenvironment. It may be a very potential anti-tumour strategy to regulate the function of Treg cells by TNFR2, thus inhibiting the tumourigenesis. The present inventors have prepared an antagonistic antibody against TNFR2, which can: 1) specifically bind to TNFR2 and block the binding of TNFR2 to the ligand TNFα thereof, thus inhibiting the proliferation of Treg cells and as a result the inhibitory function mediated thereby, and promoting the expansion of responder T cells and the anti-tumour function mediated by responder T cells and other immune cells; 2) directly mediate the killing effect on tumour cells with high TNFR2 expression, given that TNFR2 is highly expressed in human tumour cell lines; 3) have a better anti-tumour effect and form prolonged immune memory when used in combination with existing anti-PD-1/L1 antibodies. For the description of the above-mentioned antagonistic antibody against TNFR2, reference can be made to the patent application PCT/CN2020/106057, which is incorporated into the present application by reference in its entirety.

### Summary of the Invention

In the present invention, a humanized antibody against TNFR2 or an antigen-binding fragment thereof, and a pharmaceutical composition of the humanized antibody against TNFR2 or antigen-binding fragment thereof are prepared, and the use thereof is verified.

In a first aspect, the present invention provides a humanized antibody that specifically binds to TNFR2, or an antigen-binding fragment thereof, wherein the antibody or antigen-binding fragment thereof comprises a heavy chain variable region and a light chain variable region;
(1) the heavy chain variable region comprises:
   a. a VH sequence selected from any of SEQ ID NOs: 19-27,
   b. a VH sequence selected from a sequence having at least 80%, 85%, 90%, 91%, 92%, 93%, 94%, 95%, 96%, 97%, 98%, 99% or 100% sequence identity to any of SEQ ID NOs: 19-27, or,
   c. a VH sequence selected from a sequence having 1, 2, 3, 4, 5, 6, 7, 8, 9, 10 or more amino acid insertions, deletions and/or substitutions relative to any of SEQ ID NOs: 19-27;
(2) the light chain variable region comprises:
   a. a VL sequence selected from any of SEQ ID NOs: 28-37,
   b. a VL sequence selected from a sequence having at least 80%, 85%, 90%, 91%, 92%, 93%, 94%, 95%, 96%, 97%, 98%, 99% or 100% sequence identity to any of SEQ ID NOs: 28-37, or,
   c. a VL sequence selected from a sequence having 1, 2, 3, 4, 5, 6, 7, 8, 9, 10 or more amino acid insertions, deletions and/or substitutions relative to any of SEQ ID NOs: 28-37;

optionally, the amino acid insertions, deletions and/or substitutions occur in the FR region of the heavy chain variable region or/and the light chain variable region;
optionally, the substitutions are substitutions of conservative amino acids.

In certain embodiments, in the antibody or antigen-binding fragment thereof of the present invention, the heavy chain variable region comprises a VH sequence with a CDR identical to and an FR having at least 85%, 90%, 91%, 92%, 93%, 94%, 95%, 96%, 97%, 98%, 99% or 100% sequence identity to those of any of SEQ ID NOs: 19-27; the light chain variable region comprises a VL sequence with a CDR identical to and an FR having at least 85%, 90%, 91%, 92%, 93%, 94%, 95%, 96%, 97%, 98%, 99% or 100% sequence identity to those of any of SEQ ID NOs: 28-37.

In certain embodiments, the antibody or antigen-binding fragment thereof of the present invention comprises a heavy chain variable region and a light chain variable region selected from the group consisting of:
(1) VH having a sequence as shown in SEQ ID NO: 19 and VL having a sequence as shown in SEQ ID NO: 28;
(2) VH having a sequence as shown in SEQ ID NO: 20 and VL having a sequence as shown in SEQ ID NO: 29;
(3) VH having a sequence as shown in SEQ ID NO: 19 and VL having a sequence as shown in SEQ ID NO: 30;
(4) VH having a sequence as shown in SEQ ID NO: 20 and VL having a sequence as shown in SEQ ID NO: 31;
(5) VH having a sequence as shown in SEQ ID NO: 19 and VL having a sequence as shown in SEQ ID NO: 31;
(6) VH having a sequence as shown in SEQ ID NO: 21 and VL having a sequence as shown in SEQ ID NO: 32;
(7) VH having a sequence as shown in SEQ ID NO: 22 and VL having a sequence as shown in SEQ ID NO: 32;
(8) VH having a sequence as shown in SEQ ID NO: 23 and VL having a sequence as shown in SEQ ID NO: 32;
(9) VH having a sequence as shown in SEQ ID NO: 24 and VL having a sequence as shown in SEQ ID NO: 32;
(10) VH having a sequence as shown in SEQ ID NO: 25 and VL having a sequence as shown in SEQ ID NO: 32;
(11) VH having a sequence as shown in SEQ ID NO: 26 and VL having a sequence as shown in SEQ ID NO: 33;
(12) VH having a sequence as shown in SEQ ID NO: 27 and VL having a sequence as shown in SEQ ID NO: 34;
(13) VH having a sequence as shown in SEQ ID NO: 27 and VL having a sequence as shown in SEQ ID NO: 35;
(14) VH having a sequence as shown in SEQ ID NO: 27 and VL having a sequence as shown in SEQ ID NO: 33;
(15) VH having a sequence as shown in SEQ ID NO: 27 and VL having a sequence as shown in SEQ ID NO: 36;
(16) VH having a sequence as shown in SEQ ID NO: 26 and VL having a sequence as shown in SEQ ID NO: 34;
(17) VH having a sequence as shown in SEQ ID NO: 27 and VL having a sequence as shown in SEQ ID NO: 37;
(18) a combination of VH and VL having at least 80%, 85%, 90%, 91%, 92%, 93%, 94%, 95%, 96%, 97%, 98%, 99% or 100% sequence identity to a combination of sequences of any of the above (1) - (17);
(19) a combination of VH and VL having 1, 2, 3, 4, 5, 6, 7, 8, 9, 10 or more amino acid insertions, deletions and/or substitutions relative to a combination of sequences of any of the above (1) - (17); or,
(20) a combination of VH and VL with a CDR identical to and an FR having at least 85%, 90%, 91%, 92%, 93%, 94%, 95%, 96%, 97%, 98%, 99% or 100% sequence identity to those of a combination of sequences of any of the above (1) - (17);

optionally, the amino acid insertions, deletions and/or substitutions occur in the FR region of the heavy chain variable region or/and the light chain variable region;
optionally, the substitutions are substitutions of conservative amino acids.

In certain embodiments, in the antibody or antigen-binding fragment thereof of the present invention, the antibody or antigen-binding fragment thereof comprises: CDR1-VH selected from SEQ ID NO: 1, 7, or 13; CDR2-VH selected from SEQ ID NO: 2, 8, or 14; CDR3-VH selected from SEQ ID NO: 3, 9, or 15; CDR1-VL selected from SEQ ID NO: 4, 10, or 16; CDR2-VL selected from SEQ ID NO: 5, 11, or 17; and CDR3-VL selected from SEQ ID NO: 6, 12, or 18.

In certain embodiments, the antibody or antigen-binding fragment thereof of the present invention binds to human TNFR2 with a dissociation constant (KD) no more than 7 nM, and to cynomolgus monkey TNFR2 with a dissociation constant (KD) no more than 5 nM.

In certain embodiments, the antibody or antigen-binding fragment thereof of the present invention comprises or does not comprise a heavy chain constant region and/or a light chain constant region; preferably, the heavy chain constant region comprises a full-length heavy chain constant region or a fragment thereof, and the fragment can be selected from a CH1 domain, an Fc domain or a CH3 domain; preferably, the heavy chain constant region and/or the light chain constant region are/is a human heavy chain constant region and/or a human light chain constant region; preferably, the heavy chain constant region can be selected from an IgG heavy chain constant region, such as an IgG1 heavy chain constant region, an IgG2 heavy chain constant region, an IgG3 heavy chain constant region or an IgG4 heavy chain constant region; preferably, the heavy chain constant region is a human IgG1 heavy chain constant region, a human IgG2 heavy chain constant region, a human IgG3 heavy chain constant region or a human IgG4 heavy chain constant region.

In certain embodiments, the antibody or antigen-binding fragment thereof of the present invention is selected from a monoclonal antibody, a polyclonal antibody, a natural antibody, an engineered antibody, a monospecific antibody, a multispecific antibody (e.g., a bispecific antibody), a monovalent antibody, a multivalent antibody, a full-length antibody, an antibody fragment, Fab, Fab', F(ab')2, Fd, Fv, scFv, or a diabody.

In certain embodiments, the antibody or antigen-binding fragment thereof of the present invention is coupled with another molecule via or not via a linker; preferably, the another molecule can be selected from a therapeutic agent or a tracer; preferably, the therapeutic agent is selected from a radioisotope, a chemotherapeutic drug or an immunomodulator, and the tracer is selected from a radiological contrast agent, a paramagnetic ion, a metal, a fluorescent label, a chemiluminescence label, an ultrasonic contrast agent or a photosensitizer.

In certain embodiments, the antibody or antigen-binding fragment thereof of the present invention has the following properties:
(1) specifically binding to a cell expressing TNFR2 on the cell surface; (2) specifically binding to a Treg cell; (3) inhibiting the binding of TNFα to a TNFR2 protein; (4) inhibiting the binding of TNFα to TNFR2 expressed on the cell surface; (5) inhibiting the Treg proliferation and/or Treg function mediated by TNFα; (6) inhibiting the degradation of IκBα mediated by TNFα; (7) inhibiting the inhibition of Tcon cell proliferation by Treg; (8) mediating the ADCC function against a TNFR2-expressing cell; (9) increasing the ratio of CD8+ T/Treg cells in tumour infiltrating lymphocytes, TILs; or/and (10) inhibiting the tumour growth.

In a second aspect, the present invention provides a multispecific antigen-binding molecule, wherein the multispecific antigen-binding molecule comprises at least a first antigen-binding module and a second antigen-binding module, wherein the first antigen-binding module comprises the antibody or antigen-binding fragment thereof of the present invention, and the second antigen-binding module specifically binds to other antigens than TNFR2 or binds to a different TNFR2 antigenic epitope from the first antigen-binding module; preferably, the other antigens are selected from CD3, CD4, CD5, CD8, CD14, CD15, CD16, CD16A, CD19, CD20, CD21, CD22, CD23, CD25, CD33, CD37, CD38, CD40, CD40L, CD46, CD52, CD54, CD70, CD74, CD80, CD86, CD126, CD138, B7, PD-1, PD-L1, PD-2, CTLA4, PRVIG, TIGHT, HAS, CLDN18.2, MSLN, MUC, Ia, HLA-DR, tenascin, EGFR, VEGF, P1GF, ED-B fibronectin, an oncogene product, IL-2, IL-6, IL-15, IL-21, TRAII,-R1 or TRAIL-R2; preferably, the multispecific antibody is "bispecific", "trispecific" or "tetraspecific".

In a third aspect, the present invention provides a chimeric antigen receptor (CAR), wherein the chimeric antigen receptor comprises an extracellular antigen-binding domain, a transmembrane domain and an intracellular signalling domain; the extracellular antigen-binding domain comprises the humanized antibody against TNFR2 or antigen-binding fragment thereof of the first aspect of the present invention or comprises the multispecific antigen-binding molecule of the second aspect of the present invention.

In a fourth aspect, the present invention provides an immune effector cell, wherein the immune effector cell comprises the chimeric antigen receptor of the third aspect of the present invention or a nucleic acid fragment encoding the chimeric antigen receptor of the third aspect of the present invention; preferably, the immune effector cell is selected from a T cell, a natural killer (NK) cell, a natural killer T (NKT) cell, a monocyte, a macrophage, a dendritic cell or a mast cell; the T cell can be selected from an inflammatory T cell, a cytotoxic T cell, a regulatory T cell (Treg) or a helper T cell; preferably, the immune effector cell is an allogeneic immune effector cell or an autologous immune cell.

In a fifth aspect, the present invention provides an isolated nucleic acid fragment, wherein the nucleic acid fragment encodes the humanized antibody against TNFR2 or antigen-binding fragment thereof of the first aspect, the multispecific antigen-binding molecule of the second aspect or the chimeric antigen receptor of the third aspect of the present invention.

In a sixth aspect, the present invention provides a vector, wherein the vector comprises the nucleic acid fragment of the fifth aspect.

In a seventh aspect, the present invention provides a host cell, wherein the host cell comprises the vector of the sixth aspect; preferably, the cell is a prokaryotic cell or a eukaryotic cell, such as a bacterial (*Escherichia coli*) cell, a fungal (yeast) cell, an insect cell or a mammalian cell (a CHO cell line or a 293T cell line); preferably, the cell lacks a fucosyltransferase, and more preferably, the fucosyltransferase is FUT8.

In an eighth aspect, the present invention provides a method for preparing the antibody or antigen-binding fragment thereof of the present invention or the multispecific antigen-binding molecule of the present invention, comprising culturing the cell of the seventh aspect, and isolating the antibody or antigen-binding fragment thereof or the multispecific antigen-binding molecule expressed by the cell.

In a ninth aspect, the present invention provides a method for preparing an immune effector cell, comprising introducing a nucleic acid fragment encoding the aforementioned CAR into the immune effector cell, and optionally, further comprising enabling the immune effector cell to express the CAR.

In a tenth aspect, the present invention provides a pharmaceutical composition, wherein the composition comprises the antibody or antigen-binding fragment thereof of the present invention, the multispecific antigen-binding molecule of the present invention, the chimeric antigen receptor of the present invention, the immune effector cell of the present invention, the nucleic acid fragment of the present invention, the vector of the present invention, the cell of the present invention, or a product prepared from the method of the present invention; preferably, the composition further comprises a pharmaceutically acceptable carrier, a diluent or an auxiliary agent; preferably, the composition further comprises an additional anti-tumour agent, an immunotherapeutic agent or an immunosuppressant; preferably, the additional anti-tumour agent is selected from a PD-1 antibody, a PD-L1 antibody or a CTLA-4 antibody.

In an eleventh aspect, the present invention provides the use of the antibody or antigen-binding fragment thereof, the multispecific antigen-binding molecule, the chimeric antigen receptor, the immune effector cell, the nucleic acid fragment, the vector, the cell, a product prepared from the method, and the pharmaceutical composition in the preparation of a drug for treating and/or preventing diseases related to immune abnormalities; preferably, the diseases related to immune abnormalities are those related to Treg cells and/or MDSC functions; preferably, the diseases are cancers or autoimmune diseases; preferably, the cancers are selected from ovarian cancer, advanced epidermal T-cell lymphoma, stage III/IV metastatic colorectal cancer, triple-negative breast cancer, pancreatic cancer, non-small cell lung cancer, and/or advanced solid tumours resistant to CTLA-4 and PD-1 therapies, e.g., metastatic melanoma; preferably, the autoimmune diseases can be selected from rheumatoid arthritis, multiple sclerosis, systemic sclerosis, neuromyelitis optica spectrum disorder, systemic lupus erythematosus, myasthenia gravis and IgG4-related diseases.

In a twelfth aspect, the present invention provides a method for treating and/or preventing diseases related to immune abnormalities, comprising administering to a subject an effective amount of the antibody or antigen-binding fragment thereof, the multispecific antigen-binding molecule, the chimeric antigen receptor, the immune effector cell, the nucleic acid fragment, the vector, the cell, a product prepared from the method, or the pharmaceutical composition of the present invention; preferably, the diseases related to immune abnormalities are those related to Treg cells and/or MDSC functions; preferably, the diseases are cancers or autoimmune diseases; preferably, the cancers are selected from ovarian cancer, advanced epidermal T-cell lymphoma, stage III/IV metastatic colorectal cancer, triple-negative breast cancer, pancreatic cancer, non-small cell lung cancer, and/or advanced solid tumours resistant to CTLA-4 and PD-1 therapies, e.g., metastatic melanoma; preferably, the autoimmune diseases can be selected from rheumatoid arthritis, multiple sclerosis, systemic sclerosis, neuromyelitis optica spectrum disorder, systemic lupus erythematosus, myasthenia gravis and IgG4-related diseases.

In a thirteenth aspect, the present invention further provides an additional anti-tumour therapeutic agent administered to the object, such as a chemotherapeutic agent, a targeted therapeutic agent and an immunotherapeutic agent, including a PD-1/PD-L1 therapeutic agent such as an anti-PD-1/PD-L1 antibody, and an anti-CTLA-4 therapeutic agent such as an anti-CTLA-4 antibody. preferably, the additional anti-tumour therapeutic agent is selected from a PD-1/PD-L1 therapeutic agent; preferably, the PD-1/PD-L1 therapeutic agent is selected from a PD-L1 antibody.

In a fourteenth aspect, the present invention provides a method for detecting TNFR2 *in vitro,* comprising the step of contacting a sample suspected of containing TNFR2 with the antibody or antigen-binding fragment thereof of the present invention.

### Definition and Description of Terminology

Unless defined otherwise, terms used herein have the meanings as commonly understood by one of ordinary skill in the art to which the present invention belongs. For a term explicitly defined herein, the meaning of the term shall prevail from the definition.

As used herein, the term "TNFR2" refers to tumour necrosis factor receptor 2, also known as tumour necrosis factor receptor superfamily member 1B (TNFRSF1B) or CD120b, which is a membrane receptor that binds to tumour necrosis factor-α (TNFα). The TNFR2 is preferably human TNFR2.

As used herein, the terms "anti-tumour necrosis factor receptor 2 antibody", "tumour necrosis factor receptor 2 antibody", "anti-TNFR2 antibody", "TNFR2 antibody", "anti-TNFR2 antibody portion" and/or "anti-TNFR2 antibody fragment" and the like refer to any protein- or peptide-containing molecule that comprises at least a portion of an immunoglobulin molecule capable of specifically binding to TNFR2 (such as but not limited to at least one complementarity determining region (CDR) of a heavy chain or a light chain or a ligand binding portion thereof, a heavy chain variable region or a light chain variable region, a heavy chain constant region or a light chain constant region, and a framework region or any portion thereof). The TNFR2 antibody also includes an antibody-like protein scaffold (such as the tenth fibronectin type III domain (10Fn3)) containing BC, DE and FG structural loops similar in structure and solvent accessibility to CDRs of an antibody. The tertiary structure of the 10Fn3 domain is similar to that of an IgG heavy chain variable region, and those skilled in the art can graft, for example, CDRs of a TNFR2 monoclonal antibody onto a fibronectin scaffold by substituting the residues from BC, DE and FG loops of the 10Fn3 with residues from the CDR-H1, CDR-H2 or CDR-H3 region of the TNFR2 monoclonal antibody.

As used herein, the term "antibody (Ab)" refers to an immunoglobulin molecule that specifically binds to or is immunoreactive with a target antigen, including polyclonal, monoclonal, genetically engineered and other modified forms of an antibody (including but not limited to a chimeric antibody, a humanized antibody, a fully human antibody, a heterologous coupled antibody (such as bispecific, trispecific and tetraspecific antibodies, a diabody, a tribody and a tetrabody), and an antibody conjugate) and an antigen-binding fragment thereof (including, for example, Fab', F(ab')2, Fab, Fv, rIgG and scFv fragments). In addition, unless otherwise specified, the term "monoclonal antibody (mAb)" is meant to include an intact antibody molecule and an incomplete antibody fragment (e.g., Fab and F(ab')2 fragments, which lack the Fc fragment of an intact antibody (cleared from animal circulation more quickly) and therefore lack an Fc-mediated effector function) capable of specifically binding to a target protein (see Wahl et al., J.Nucl.Med.24:316,1983; the content of which is incorporated herein by reference).

As used herein, the term "monoclonal antibody" refers to an antibody derived from a single clone (including any eukaryotic, prokaryotic or bacteriophage clone), without limitation by the method by which the antibody is produced.

As used herein, the terms "antigen-binding fragment" and "antibody fragment" are interchangeable and refer to one or more antibody fragments that retain the ability to specifically bind to a target antigen. The antigen-binding function of an antibody can be performed by a fragment of a full-length antibody. An antibody fragment can be Fab, F(ab')2, scFv, SMIP, a diabody, a tribody, an affibody, a nanobody, an aptamer or a domain antibody. Examples of binding fragments encompassed by the term "antigen-binding fragment" of an antibody include, but are not limited to: (i) an Fab fragment, a monovalent fragment consisting of VL, VH, CL and CH1 domains; (ii) an F(ab)2 fragment, a bivalent fragment comprising two Fab fragments linked by a disulphide bond in a hinge region; (iii) an Fd fragment consisting of VH and CH1 domains; (iv) an Fv fragment consisting of VL and VH domains of a single arm of an antibody; (v) dAb comprising VH and VL domains; (vi) a dAb fragment consisting of a VH domain (Ward et al., Nature 341:544-546,1989); (vii) dAb consisting of a VH or VL domain; (viii) an isolated complementarity determining region (CDR); and (ix) a combination of two or more isolated CDRs which may optionally be linked via a synthetic linker. In addition, although two domains VL and VH of an Fv fragment are encoded by independent genes, these two domains can be joined via a linker using a recombination method, wherein the linker enables the preparation of a single protein chain in which the VL and VH regions are paired to form a monovalent molecule (known as single chain Fv (scFv); see, for example, Bird et al., Science 242:423-426, 1988 and Huston et al., Proc. Natl. Acad. Sci. USA 85:5879-5883, 1988). These antibody fragments can be obtained using conventional techniques known to those skilled in the art, and these fragments are screened for use in the same way as an intact antibody. An antigen-binding fragment can be generated by a recombinant DNA technology, by the enzymatic or chemical cleavage of an intact immunoglobulin, or in some embodiments by a chemical peptide synthesis procedure known in the art.

As used herein, the term "complementarity determining region (CDR)" refers to a hypervariable region found in both light chain and heavy chain variable domains. The more conservative portion of a variable domain is called the framework region (FR). As understood in the art, the amino acid positions representing the hypervariable region of an antibody may vary according to the context and various definitions known in the art. Some positions within a variable domain can be regarded as hybrid hypervariable positions, as these positions can be regarded as being within the hypervariable regions under a set of standards (such as IMGT or KABAT) and outside the hypervariable regions under a different set of standards (such as KABAT or IMGT). One or more of these positions may also be found in an extended hypervariable region. The present invention includes an antibody comprising modifications in these hybrid hypervariable positions. The variable domains of a natural heavy chain and light chain each comprise four framework regions predominantly in a sheet configuration, which are linked by three CDRs (CDR1, CDR2 and CDR3) that form a loop linking the sheet structure, and in some cases form part of the sheet structure. The CDRs in each chain are closely held together in order of FR1-CDR1-FR2-CDR2-FR3-CDR3-FR4 by the FR region, and together with the CDRs from other antibody chains, contribute to the formation of an antigen-binding site of an antibody (see Kabat et al., Sequences of Proteins of Immunological Interest, National Institute of Health, Bethesda, Md.1987; which is incorporated herein by reference). For example, in the present invention, CDR1-VH, CDR2-VH and CDR3-VH refer to the first CDR, the second CDR and the third CDR of a heavy chain variable region (VH), respectively, and these three CDRs constitute the CDR combination of a heavy chain (or a variable region thereof) (the VHCDR combination); CDR1-VL, CDR2-VL and CDR3-VL refer to the first CDR, the second CDR and the third CDR of a light chain variable region (VL), respectively, and these three CDRs constitute the CDR combination of a light chain (or a variable region thereof) (the VLCDR combination).

As used herein, the term "Kabat numbering system" generally refers to the immunoglobulin alignment and numbering system proposed by Elvin A.Kabat (see, for example, Kabat et al., Sequences of Proteins of Immunological Interest, 5th Ed. Public Health Service, National Institutes of Health, Bethesda, Md., 1991).

As used herein, the term "VH" refers to a variable region of an antibody immunoglobulin heavy chain (including a heavy chain of Fv, scFv or Fab). The term "VL" refers to a variable region of an immunoglobulin light chain (including a light chain of Fv, scFv, dsFv or Fab).

The term "heavy chain constant region" herein refers to the carboxyl terminus portion of an antibody heavy chain, which is not directly involved in the binding of an antibody to an antigen, but exhibits an effector function, such as an interaction with an Fc receptor, and has a more conservative amino acid sequence relative to the variable domain of an antibody. The "heavy chain constant region" comprises at least: a CH1 domain, a hinge region, a CH2 domain, a CH3 domain, or variants or fragments thereof. A "heavy chain constant region" includes a "full-length heavy chain constant region" and a "heavy chain constant region fragment", the former has a structure substantially similar to that of a natural antibody constant region, while the latter only includes "a portion of the full-length heavy chain constant region". Exemplarily, a typical "full-length antibody heavy chain constant region" consists of CH1 domain-hinge region-CH2 domain-CH3 domain, which also includes a CH4 domain when an IgE antibody is referred to, and does not include the CH1 domain when a heavy chain antibody is referred to. Exemplarily, a typical "heavy chain constant region fragment" can be selected from CH1, Fc or CH3 domains.

The term "light chain constant region" herein refers to the carboxyl terminus portion of an antibody light chain, which is not directly involved in the binding of an antibody to an antigen, and the light chain constant region can be selected from a constant κ domain or a constant λ domain.

The term "Fc" herein refers to the carboxyl terminus portion of an intact antibody obtained by hydrolysing the antibody with papain, which typically comprises CH3 and CH2 domains of an antibody. The Fc region includes, for example, an Fc region of a natural sequence, a recombinant Fc region and a variant Fc region. Although the boundary of the Fc region of an immunoglobulin heavy chain can be slightly changed, the Fc region of a human IgG heavy chain is usually defined as the region extending from the amino acid residue at position Cys226 or from Pro230 to its carboxyl terminus. The C-terminal lysine of the Fc region (residue 447 according to the EU Kabat numbering system) can be removed, for example, during the production or purification of an antibody, or by recombinant engineering of a nucleic acid encoding an antibody heavy chain, so the Fc region may or may not include Lys447.

As used herein, the terms "percent (%) sequence identity" and "percent (%) sequence consistency" are interchangeable, and refer to the percentage of amino acid (or nucleotide) residues of a candidate sequence that are identical to those of a reference sequence upon aligning sequences and introducing gaps (if necessary) in order to achieve maximum percent sequence identity (for example, gaps can be introduced in one or both of the candidate and reference sequences for optimal alignment, and non-homologous sequences can be ignored for comparison purposes). For the purpose of determining the percent sequence identity, alignment can be achieved in a variety of ways well known to those skilled in the art, for example, using publicly available computer software, such as BLAST, ALIGN or Megalign (DNASTAIi) software. Those skilled in the art can determine appropriate parameters for measuring alignment, including any algorithm required to achieve maximum alignment over the full-length range of the sequences aligned. For example, a reference sequence aligned for comparison with a candidate sequence can show that the candidate sequence exhibits a sequence consistency from 50% to 100% over the full length of the candidate sequence or a selected portion of consecutive amino acid (or nucleotide) residues of the candidate sequence. The length of a candidate sequence aligned for comparison purposes may be, for example, at least 30% (e.g., 30%, 40%, 50%, 60%, 70%, 80%, 90% or 100%) of the length of a reference sequence. When a position in a candidate sequence is occupied by the same amino acid (or nucleotide) residue as in the corresponding position in a reference sequence, these molecules are identical at that position.

The term "conservative amino acids" herein generally refers to amino acids that belong to the same class or have similar characteristics (such as charge, side chain size, hydrophobicity, hydrophilicity, backbone conformation and rigidity). Exemplarily, the following amino acids in each group are each other's conservative amino acid residues, and a substitution of amino acid residues in the group is a substitution of conservative amino acids:
(1) acidic amino acids: Asp (D) and Glu (E);
(2) basic amino acids: Lys (K), Arg (R) and His (H);
(3) hydrophilic uncharged amino acids: Ser (S), Thr (T), Asn (N) and Gln (Q);
(4) aliphatic uncharged amino acids: Gly (G), Ala (A), Val (V), Leu (L) and Ile (I);
(5) nonpolar uncharged amino acids: Cys (C), Met (M) and Pro (P);
(6) aromatic amino acids: Phe (F), Tyr (Y) and Trp (W).

As used herein, the term "specific binding" refers to a binding reaction that determines the presence of an antigen in a heterogeneous population of a protein and other biomolecules which are specifically recognized by antibodies or antigen-binding fragments thereof, for example. An antibody or an antigen-binding fragment thereof that specifically binds to an antigen will bind to the antigen with a KD of less than 100 nM. For example, an antibody or an antigen-binding fragment thereof that specifically binds to an antigen will bind to the antigen with a KD of up to 100 nM (for example, between 1 pM and 100 nM). An antibody or an antigen-binding fragment thereof that does not show a specific binding to a specific antigen or epitope thereof will show a KD greater than 100 nM (for example, greater than 500 nM, 1 µM, 100 µM, 500 µM or 1 mM) for the specific antigen or epitope thereof. A variety of immunoassays can be used to select antibodies that are specifically immunoreactive with specific proteins or carbohydrates. For example, a solid-phase ELISA immunoassay is conventionally used to select antibodies that are specifically immunoreactive with proteins or carbohydrates. See Harlow & Lane, Antibodies, A Laboratory Manual, Cold Spring Harbour Press, New York (1988) and Harlow & Lane, Using Antibodies, A Laboratory Manual, Cold Spring Harbour Press, New York (1999), which describe immunoassays and conditions that can be used to determine the specific immunoreactivity.

As used herein, the term "multispecific antibody" refers to an antibody having at least two antigen-binding sites, each of which binds to a different epitope of the same antigen or to a different epitope of a different antigen. Therefore, terms such as "bispecific", "trispecific" and "tetraspecific" refer to the number of different epitopes to which an antibody/antigen-binding molecule can bind. The term "bispecific antibody" refers to an antibody with monoclonal binding specificities to at least two different antigens, which is generally a human or humanized antibody. In the present invention, one of the binding specificities can be detected against an antigenic epitope of TNFR2, and the other can be detected against another antigenic epitope of TNFR2 or any other antigen, such as against a cell surface protein, a receptor, a receptor subunit, a tissue-specific antigen, a virus-derived protein, a virus-encoded envelope protein, a bacterium-derived protein or a bacterial surface protein.

As used herein, the term "valence" means the presence of a specified number of binding sites in an antibody/antigen-binding molecule. Therefore, the terms "monovalent", "bivalent", "tetravalent" and "hexavalent" indicate the presence of one binding site, two binding sites, four binding sites and six binding sites in an antibody/antigen-binding molecule, respectively.

As used herein, the term "humanized antibody" refers to a non-human antibody that has been genetically engineered, with amino acid sequences modified to improve the homology with the sequences of a human antibody. Generally speaking, all or part of the CDR regions of a humanized antibody come from a non-human antibody (a donor antibody), and all or part of the non-CDR regions (for example, a variable region FR and/or a constant region) come from a human immunoglobulin (a receptor antibody). A humanized antibody generally retains or partially retains the expected properties of a donor antibody, including but not limited to antigen specificity, affinity, reactivity, ability to improve immune cell activities, ability to enhance immune responses, etc.

As used herein, the term "chimeric antibody" refers to an antibody that has a variable sequence of an immunoglobulin from a source organism (such as a rat or a mouse) and a constant region of an immunoglobulin from a different organism (such as human). Methods for producing a chimeric antibody are known in the art. See, for example, Morrison, 1985, Science 229(4719) : 1202-7; Oi et al., 1986, Bio Techniques 4:214-221; and Gillies et al., 1985 J Immunol Methods 125:191-202; which are incorporated herein by reference.

As used herein, the term "antibody conjugate" refers to a couplet/conjugate formed by chemical bonding of an antibody molecule to another molecule directly or via a linker. The another molecule can be a therapeutic agent or a tracer; preferably, the therapeutic agent is selected from a radioisotope, a chemotherapeutic drug or an immunomodulator, and the tracer is selected from a radiological contrast agent, a paramagnetic ion, a metal, a fluorescent label, a chemiluminescence label, an ultrasonic contrast agent or a photosensitizer. An "antibody conjugate" is, for example, an antibody-drug conjugate (ADC), in which the drug molecule is the another molecule.

The term "antigen chimeric receptor (CAR)" herein refers to an artificial immune effector cell surface receptor engineered to express on immune effector cells and specifically bind to antigens, comprising at least (1) an extracellular antigen-binding domain, such as a variable heavy chain or light chain of an antibody, (2) a transmembrane domain anchoring the CAR into an immune effector cell, and (3) an intracellular signalling domain. The CAR can redirect T cells and other immune effector cells to selected targets, such as cancer cells, in a non-MHC-restricted way by using an extracellular antigen-binding domain.

As used herein, the term "regulatory T cells" or "Tregs", also formerly known as suppressor T cells, refers to a population of lymphocytes that negatively regulate immune responses of the body, so as to maintain resistance to autoantigens, control excessive immune responses, avoid immune damage to normal cells and prevent the occurrence of autoimmune diseases. Tregs express the following biomarkers: CD4, FOXP3 and CD25, which are considered to be derived from the same lineage as naive CD47 cells. Tregs play an extremely important role in tumourigenesis. A large number of studies have shown that the number of Treg cells in the tumour microenvironment increases significantly, including melanoma, ovarian cancer, breast cancer, colon cancer, lung cancer, pancreatic cancer, etc., and the number of Treg cells is closely related to the survival rate of tumour patients. In addition, tumour cells can induce the proliferation of tumour infiltrating Treg cells, and the proliferating Treg cells will secrete a large number of immunosuppressive factors such as TGF-β to inhibit the functions of immune cells such as CD8+T cells and finally hinder the killing effect of immune cells on tumours, which constitutes an important drug resistance mechanism for the failure of immunotherapies in various solid tumours and haematological tumours. Recent studies have shown that the immunological tolerance of patients treated with immunotherapies such as PD-1/PD-L1 is also closely related to Tregs.

As used herein, the term "vector" includes nucleic acid vectors, such as DNA vectors (e.g., plasmids), RNA vectors, viruses or other suitable replicons (e.g., viral vectors). A variety of vectors have been developed for delivering a polynucleotide encoding an exogenous protein to a prokaryotic or eukaryotic cell. The expression vector of the present invention contains polynucleotide sequences and additional sequence elements, for example, for expressing proteins and/or integrating these polynucleotide sequences into the genome of mammalian cells. Certain vectors that can be used to express the antibodies and antibody fragments of the present invention include plasmids containing regulatory sequences (such as promoters and enhancer regions) that direct gene transcription. Other useful vectors for expressing antibodies and antibody fragments contain polynucleotide sequences that enhance the translation rate of these genes or improve the stability or nuclear export of mRNA produced by gene transcription. These sequence elements include, for example, 5' and 3' untranslated regions, internal ribosome entry sites (IRESs) and polyadenylation signal sites, so as to direct the effective transcription of genes carried on expression vectors. The expression vector of the present invention may also contain a polynucleotide encoding a marker for selection of a cell containing such a vector. Examples of suitable markers include genes encoding resistance to antibiotics such as ampicillin, chloramphenicol, kanamycin or nourseothricin.

As used herein, the terms "subject", "object" and "patient" refer to an organism treated for a specific disease or disorder (such as a cancer or an infectious disease) as described herein. Examples of objects and patients include mammals treated for diseases or disorders (for example, cell proliferative disorders such as cancers or infectious diseases), such as humans, primates, pigs, goats, rabbits, hamsters, cats, dogs, guinea pigs, members of the family *Bovidae* (such as cattle, bison, buffalo, elk and yak), cows, sheep, horses and bisons.

As used herein, the term "treatment" refers to a surgical or therapeutic treatment with an aim to prevent and slow down (reduce) unwanted physiological changes or lesions in an object to be treated, such as the progression of cell proliferative disorders (such as cancers or infectious diseases). Beneficial or desired clinical outcomes include, but are not limited to, the alleviation of symptoms, the weakening of disease severity, the stabilization of disease state (i.e., no deterioration), the delay or slowing down of disease progression, the amelioration or mitigation of disease state, and remission (whether partial or complete), whether detectable or undetectable. Objects in need of treatment include those who already have a disorder or disease, those who are susceptible to a disorder or disease or those who intend to prevent a disorder or disease. When terms such as slowing down, alleviation, weakening, mitigation, and remission are referred to, their meanings also include elimination, disappearance, non-occurrence and other circumstances.

As used herein, the term "effective amount" refers to an amount of a therapeutic agent that is effective in preventing or relieving a disease symptom or the progression of the disease when administered alone or in combination with another therapeutic agent to a cell, tissue or subject. The "effective amount" also refers to an amount of a compound sufficient to relieve symptoms, such as treating, curing, preventing or relieving related medical disorders, or increasing the speed of treating, curing, preventing or relieving these disorders. When an active ingredient is administered alone to an individual, a therapeutically effective dose refers only to the dosage of the ingredient. When a certain combination is applied, a therapeutically effective dose refers to the combined dosage of the active ingredients that produce a therapeutic effect, whether administered in combination, sequentially or simultaneously.

### Brief Description of the Drawings

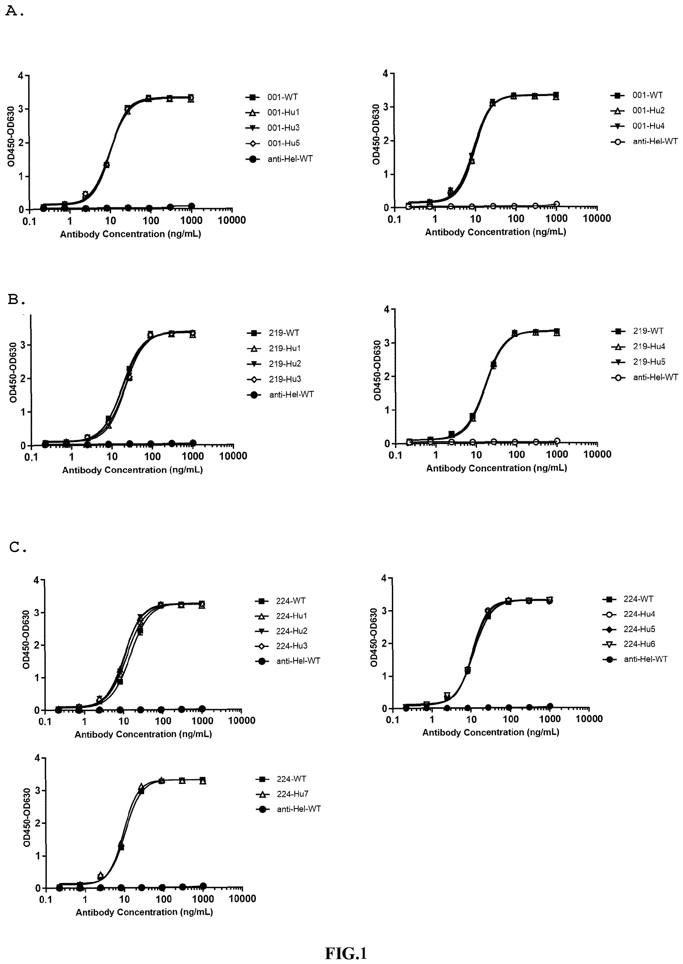

FIG. 1 shows the binding ability of test antibodies to a recombinant human TNFR2 protein. FIGs. A, B, and C show the binding ability of humanized molecules of test antibodies #001, #219, and #224 to the recombinant human TNFR2 protein, respectively; WT is a human-murine chimeric wild-type molecule, and the anti-Hel-WT isotype control antibody is a negative control of this experiment.

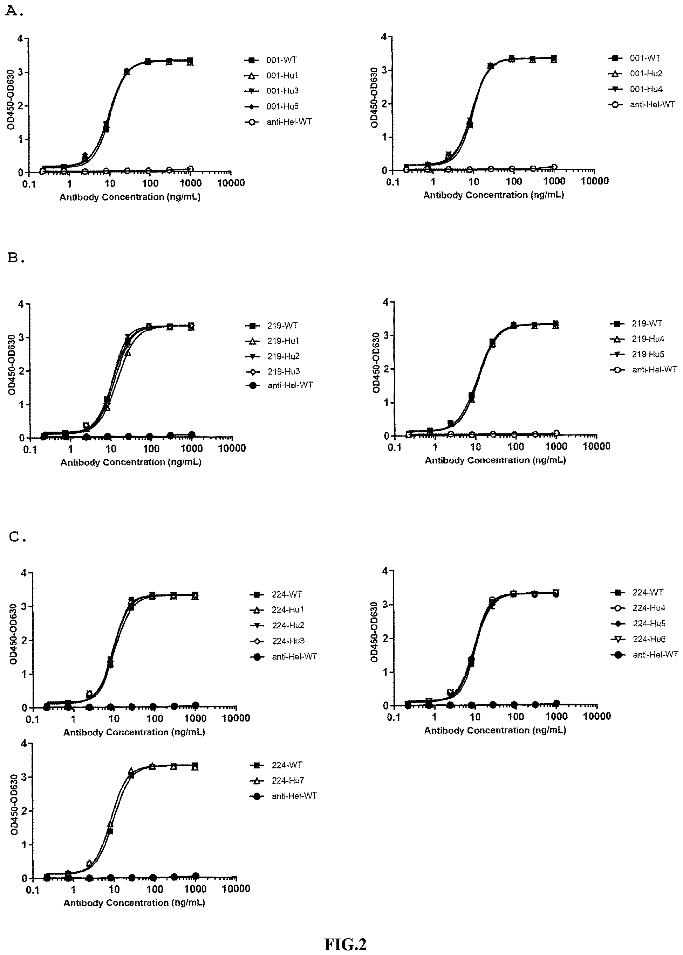

FIG. 2 shows the binding ability of test antibodies to a recombinant cynomolgus monkey TNFR2 protein. FIGs. A, B, and C show the binding ability of humanized molecules of test antibodies #001, #219, and #224 to the recombinant cynomolgus monkey TNFR2 protein, respectively; WT is a human-murine chimeric wild-type molecule, and the anti-Hel-WT isotype control antibody is a negative control of this experiment.

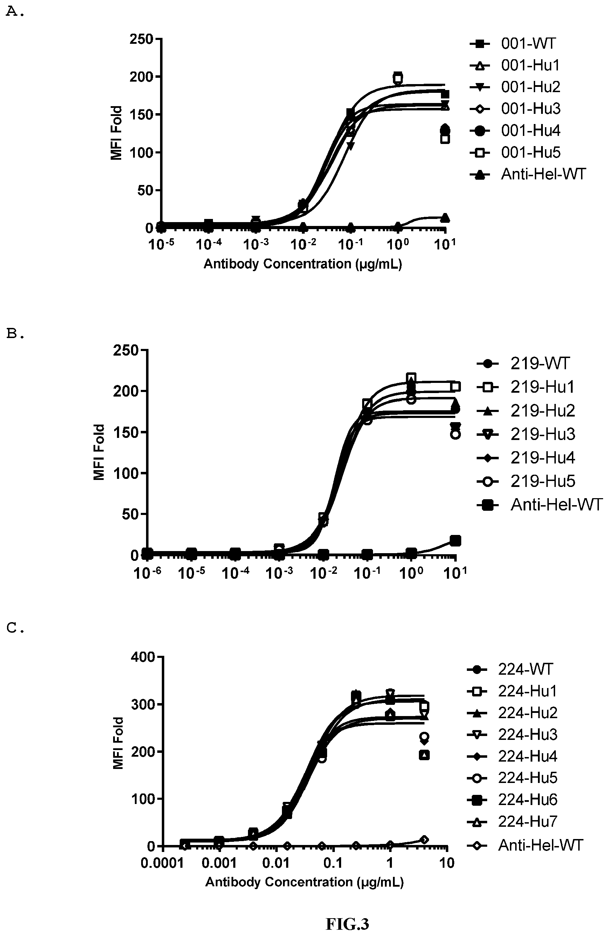

FIG. 3 shows the binding ability of test antibodies to CHO-TNFR2 cells highly expressing human TNFR2. FIGs. A, B, and C show the binding ability of humanized molecules of test antibodies #001, #219, and #224 to the CHO-TNFR2 cells, respectively; the anti-Hel-WT is a negative isotype control antibody.

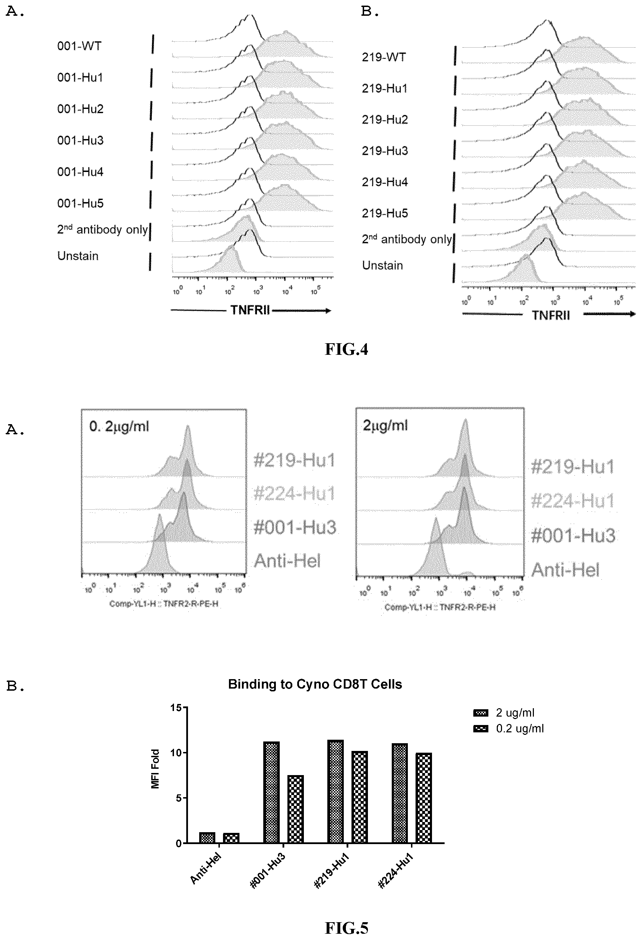

FIG. 4 shows the binding ability of test antibodies to Treg cells. FIGs. A and B are overlapping diagrams showing binding of humanized molecules of test antibodies #001 and #219 to the Treg cells, respectively. The hollow peak is a diagram showing binding of the anti-Hel-WT negative isotype control antibody, and the solid peak is a diagram showing binding of each antibody to the Treg cells; the groups "only secondary antibody" and "unstained" (a blank cell control without any antibody) are both negative controls, and the antibody concentration is 200 ng/ml.
FIG. 5 shows the binding of test antibodies to the activated CD8+ T cells in the cynomolgus monkey PBMC. FIG. A is an overlapping diagram showing binding of test antibodies at concentrations of 2 µg/ml and 0.2 µg/ml, respectively, to the cynomolgus monkey CD8+ T cells. FIG. B shows the mean fluorescence intensity fold of test antibodies at concentrations of 2 µg/ml and 0.2 µg/ml, respectively, binding to the cynomolgus monkey CD8+ T cells (the ratio of the mean fluorescence intensity of binding of the test samples to the mean fluorescence intensity of that of the sample with only secondary antibody). The anti-Hel isotype control antibody is a negative control of this experiment.

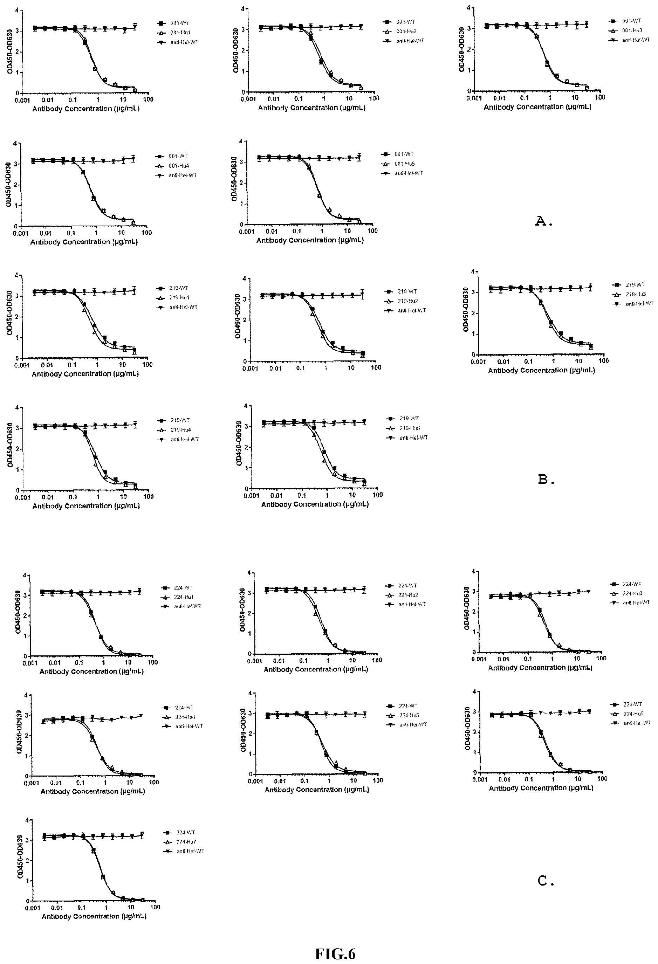

FIG. 6 shows the inhibition of interaction between human TNFα and a recombinant human TNFR2 protein by test antibodies. FIGs. A, B, and C show the inhibition of TNFα-TNFR2 by humanized molecules of test antibodies #001, #219, and #224, respectively; the anti-Hel-WT isotype control antibody is a negative control of this experiment.

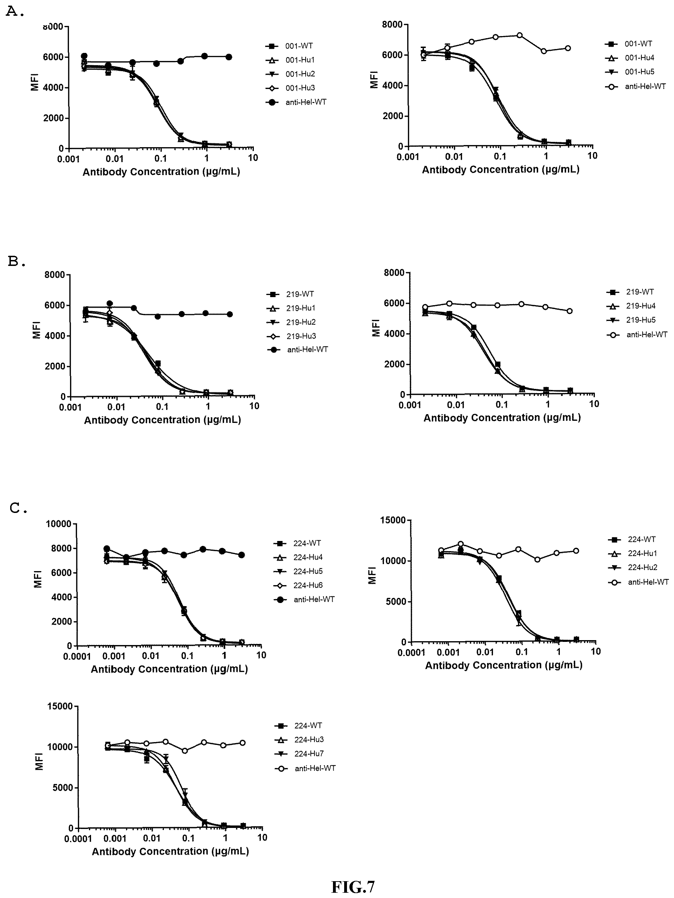

FIG. 7 shows the inhibition of interaction between human TNFα and human TNFR2 expressed by CHO-TNFR2 cells by test antibodies. FIGs. A, B, and C show the inhibition of interaction between TNFα and CHO-TNFR2 by humanized molecules of test antibodies #001, #219, and #224, respectively; the anti-Hel-WT is a negative isotype control antibody.

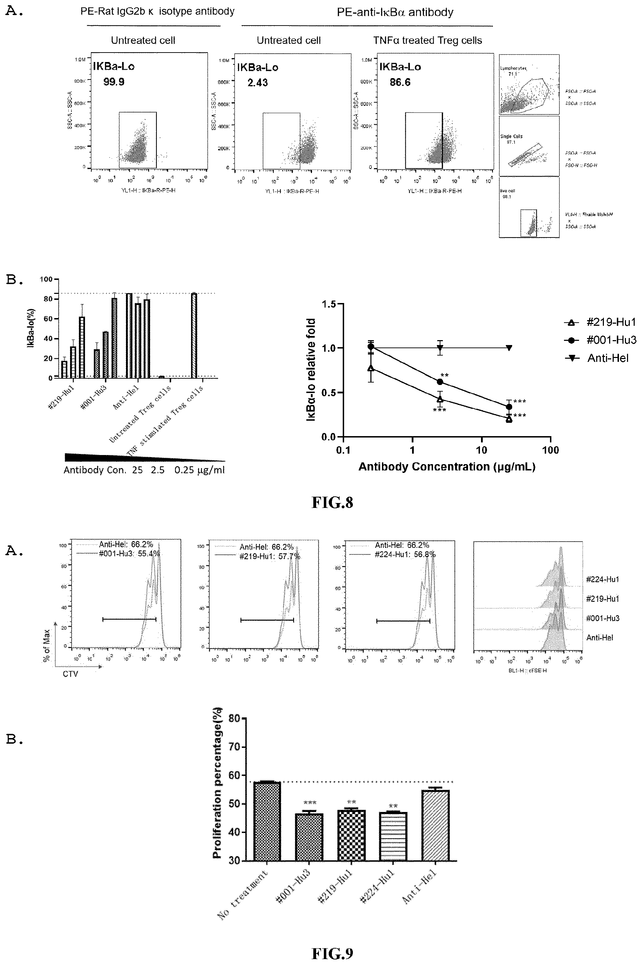

FIG. 8 shows the inhibitory effect of test antibodies on TNFα-induced degradation of IκBα. FIG. A shows the gating strategy of this experiment. FIG. B shows the inhibitory effect of test antibodies on TNFα-induced degradation of IκBα; the left-hand panel shows a histogram of the percentage of antibodies at different concentrations to IκBα^{low}, and the right-hand panel shows a diagram of the change of antibody concentration gradients versus fold of IκBα^{low} percentage relative to the negative control antibody anti-Hel. The test concentrations of antibodies are 0.25, 2.5 and 25 µg/ml, respectively; "non-treated Treg cells" mean Treg cells without any treatment, and TNF means Treg cells treated with 10 ng/ml TNFα; Rat IgG2b-PE is the negative isotype control (anti-Hel) of the Anti-IκBα-PE antibody. There is a statistical difference between test antibodies at each concentration and the negative isotype control anti-Hel (** p < 0.01, ***p < 0.001, Two-Way ANOVA Analysis).
FIG. 9 shows the inhibition of TNFα-induced proliferation of Treg cells by test antibodies. FIG. A is an overlapping diagram showing the effect of different test antibodies and the negative isotype control anti-Hel on proliferation of Tregs, in which the dotted line represents the anti-Hel and the solid line represents test antibodies. FIG. B shows the inhibitory effect of different antibodies on TNFα-induced proliferation of Tregs, in which the ordinate represents the proliferation percentage of Tregs, and the abscissa represents test antibodies. The groups "no drug treatment" (that is, without any drug treatment) and anti-Hel are negative controls of this experimental system. The experimental concentration of each antibody is 12.5 µg/ml. There is a statistical difference between each test antibody and the negative isotype control anti-Hel (**p < 0.01, ***p < 0.001, One-Way ANOVA Analysis).

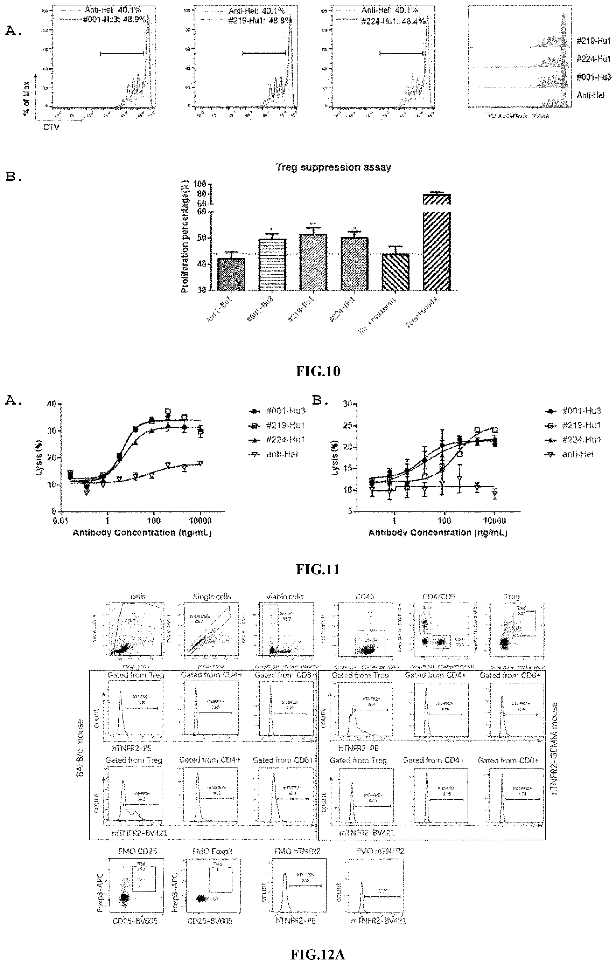

FIG. 10 shows the inhibitory effect of test antibodies on the inhibitory activity of Tcon cell proliferation by Tregs. FIG. A is an overlapping diagram showing the effect of different test antibodies and the negative isotype control anti-Hel on the inhibition of Tcon cell proliferation by Tregs, in which the dotted line represents the anti-Hel, and the solid line represents test antibodies. FIG. B shows the inhibitory effect of different antibodies on the inhibition of Tcon cell proliferation by Tregs, in which the ordinate represents the proliferation percentage of Tcon cells, and the abscissa represents test antibodies. The groups "no drug treatment" (i.e., No treat) and Anti-Hel are negative controls of this experimental system, while the group Tcon+beads represents the proliferation of Tcon in the absence of Treg cells, which is a positive control of the experimental system. The experimental concentration of each antibody is 12.5 µg/ml. There is a statistical difference between each test antibody and the negative isotype control anti-Hel (*p < 0.05, **p < 0.01, One-Way ANOVA Analysis).
FIG. 11 shows the ADCC killing effect of test antibodies. FIG. A shows the ADCC killing effect of test antibodies on Tregs. FIG. B shows the ADCC killing effect of test antibodies on CHO-TNFR2 cells highly expressing human TNFR2. The anti-Hel is a negative control antibody.

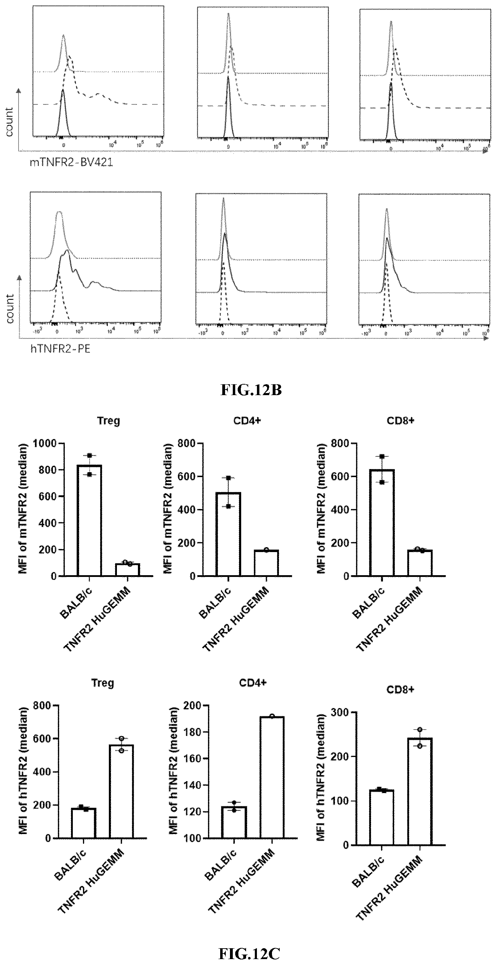

FIGs. 12A-12C show the identification of expression of hTNFR2 and mTNFR2 in immune cells in the blood of hTNFR2 transgenic mice (TNFR2 HuGEMM). FIG. 12A shows the FACS gating strategy of immune cells in the blood of TNFR2 HuGEMM (also known as hTNFR2-GEMM) mice, the expression level of mTNFR2 and hTNFR2 in gated CD4+ T cells, CD8+ T cells and Treg cells, and gated Fluorescence Minus One (FMO) controls. FIG. 12B shows the peak plots of expression level of mTNFR2 and hTNFR2 in CD4+ T cells, CD8+ T cells and Treg cells in the blood of TNFR2 HuGEMM and Balb/c wild-type mice; dash dot line: isotype, dotted line: BALB/c mouse, solid line: TNFR2 HuGEMM. FIG. 12C shows the statistical diagrams about the mean fluorescence intensity of mTNFR2 and hTNFR2 in CD4+ T cells, CD8+ T cells and Treg cells in the blood of TNFR2 HuGEMM and Balb/c wild-type mice.

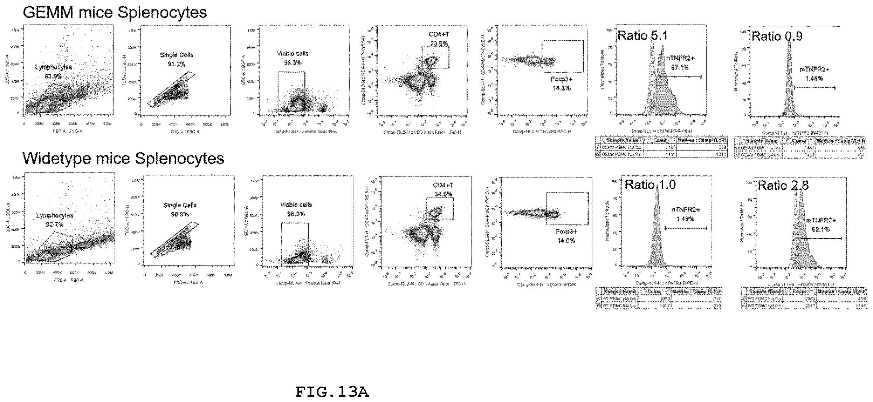

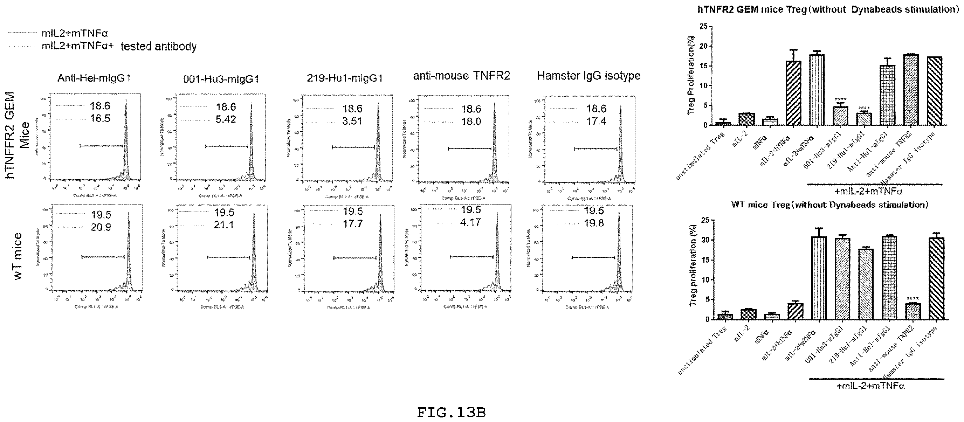

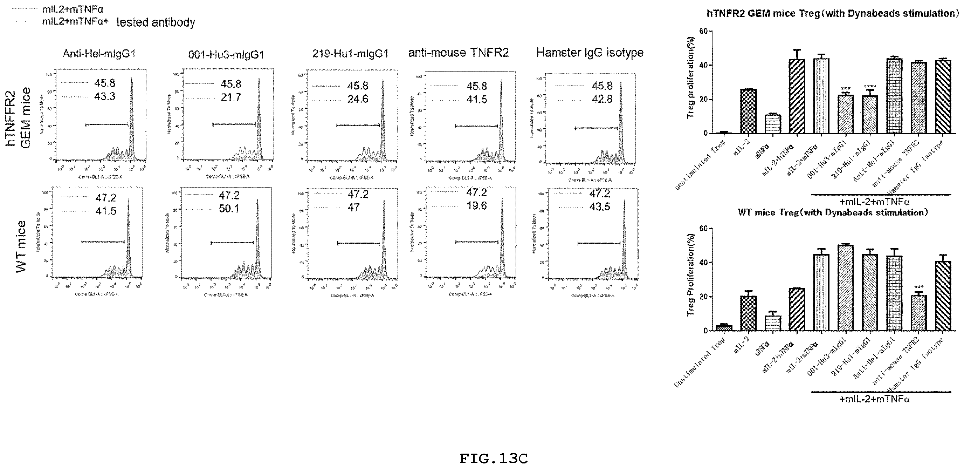

FIGs. 13A-13C show the inhibition of mTNFα-induced proliferation of Treg cells in TNFR2 HuGEMM (referred to as GEMM for short in the figures) mice by TNFR2 antibodies. FIG. 13A shows the level of human TNFR2 and mouse TNFR2 expressed by Treg cells in the spleens of a GEMM mouse and a wild-type WT Balb/c mouse. FIGs. 13B and 13C show the inhibitory effect of test TNFR2 antibodies on mTNFα-induced proliferation of Treg cells in a GEMM mouse or a WT Balb/c mouse without activator (B) or with activator (C) Dynabeads mouse T-activator CD3/CD28. The experimental concentration of each antibody is 10 µg/ml. There is a statistical difference between each test antibody and the negative isotype control (***p < 0.001, ****p < 0.0001, One-Way ANOVA Analysis).

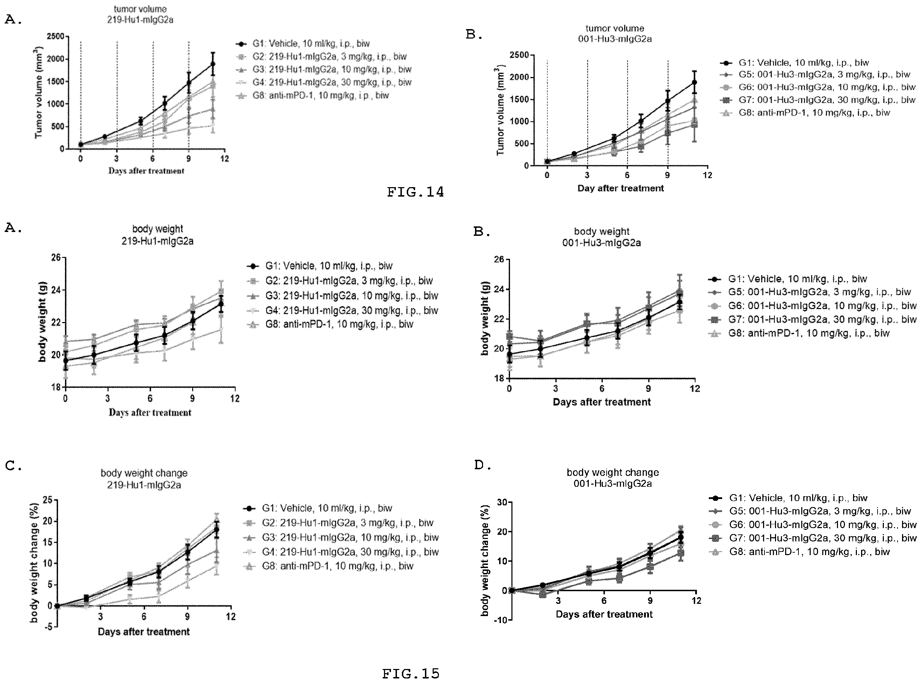

FIG. 14 shows that an anti-TNFR2 antibody can inhibit the tumour growth in a TNFR2 HuGEMM mouse in a dose-dependent manner. FIGs. A and B show that 219-Hu1-mIgG2a and 001-Hu3-mIgG2a at doses of 30 mg/kg, 10 mg/kg, and 3 mg/kg can inhibit the growth of CT26-hTNFR2 KI tumour in a dose-dependent manner compared with the vehicle control group, respectively.
FIG. 15 shows that an anti-TNFR2 antibody inhibits the tumour growth in a TNFR2 HuGEMM mouse without causing weight loss in the mouse. FIGs. A-B show the changes in absolute body weight of mice after dosing of different doses of 219-Hu1-mIgG2a or 001-Hu3-mIgG2a. FIGs. C-D show the change rate of body weight of mice after dosing of different doses of 219-Hu1-mIgG2a or 001-Hu3-mIgG2a.

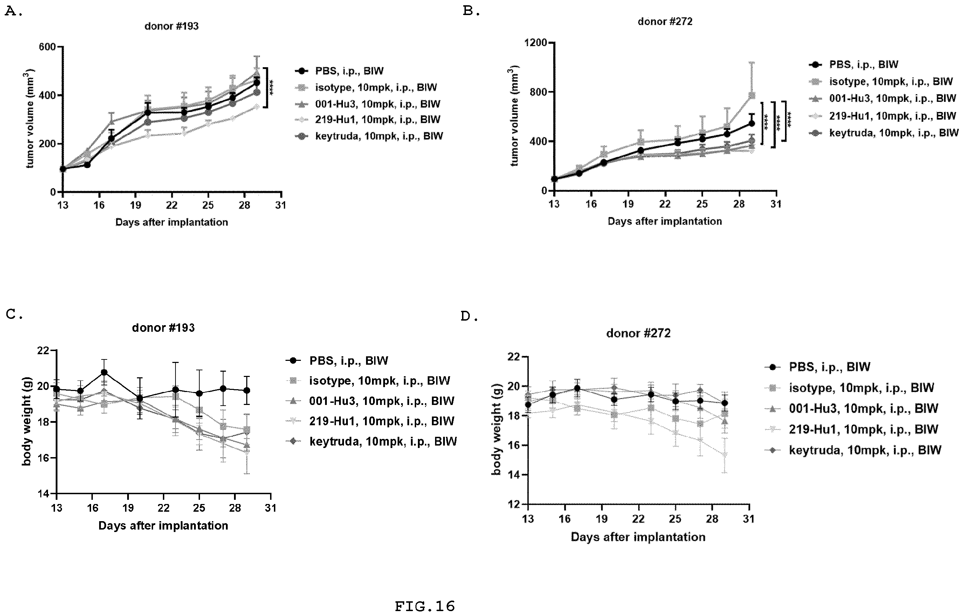

FIG. 16 shows that an anti-TNFR2 antibody can significantly inhibit the tumour growth in two different PBMC donors in a humanized mouse colon cancer tumour model with immune reconstruction of human PBMCs. FIG. A shows that in donor #193, the anti-TNFR2 antibody 219-Hu1 has a significant efficacy. FIG. B shows that in donor #272, on Day 29 after the tumour inoculation, compared with the control antibody group, the groups of 001-Hu3, 219-Hu1, and Keytruda all have tumour growth significantly inhibited, and there is a statistical difference (****: p < 0.0001, vs. isotype, Two way ANOVA). FIGs. C-D show that in donor #193 and donor #272, both the control group and the dosing groups show weight loss of mice.

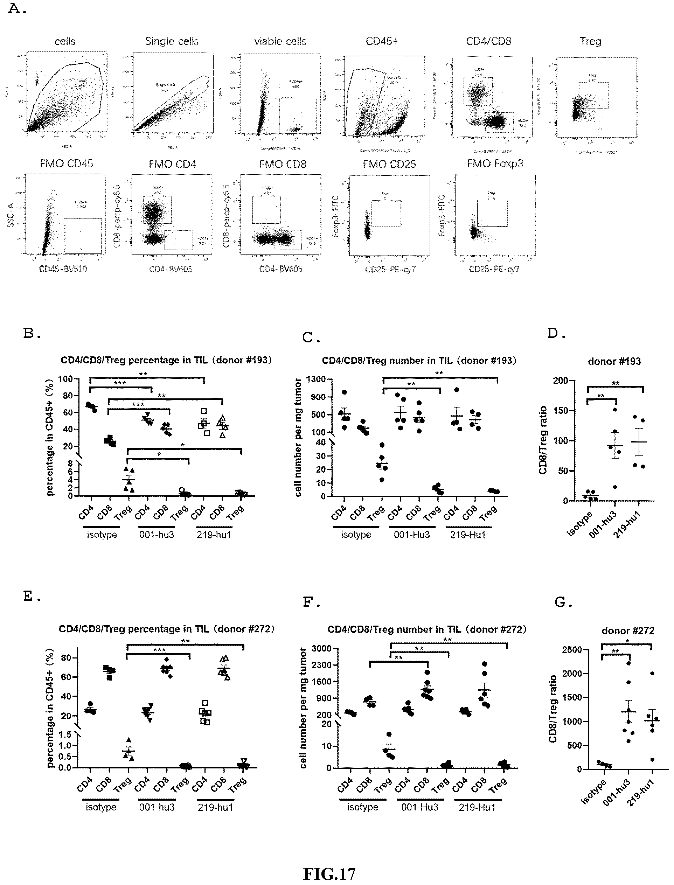

FIG. 17 shows that an anti-TNFR2 antibody can significantly increase the ratio of CD8+ T/Treg cells in tumour infiltrating lymphocytes, TILs. FIG. A shows the gating strategy of CD4+ T, CD8+ T and Treg cells in tumour infiltrating lymphocytes, TILs. FIG. B shows the proportion of CD4+ T, CD8+ T and Treg in CD45+ cells of tumour infiltrating lymphocytes (TILs) after treatment with the isotype antibody, the 001-Hu3 antibody and the 219-Hu1 antibody in donor #193. FIG. C shows the absolute number of CD4+ T, CD8+ T and Treg in TILs per mg of tumour tissue after treatment with the isotype antibody, the 001-Hu3 antibody and the 219-Hu1 antibody in donor #193. FIG. D shows the ratio of CD8+ T/Treg in TILs after treatment with the isotype antibody, the 001-Hu3 antibody and the 219-Hu1 antibody in donor #193. FIG. E shows the proportion of CD4+ T, CD8+ T and Treg in CD45+ cells of TILs after treatment with the isotype antibody, the 001-Hu3 antibody and the 219-Hu1 antibody in donor #272. FIG. 17F shows the absolute number of CD4+ T, CD8+ T and Treg in TILs per g of tumour tissue after treatment with the isotype antibody, the 001-Hu3 antibody and the 219-Hu1 antibody in donor #272. FIG. 17G shows the ratio of CD8+ T/Treg in tumour infiltrating lymphocytes, TILs after treatment with the isotype antibody, the 001-Hu3 antibody and the 219-Hu1 antibody in donor #272 (* : p < 0.05, **: p < 0.01, ***: p < 0.001, unpaired T test).

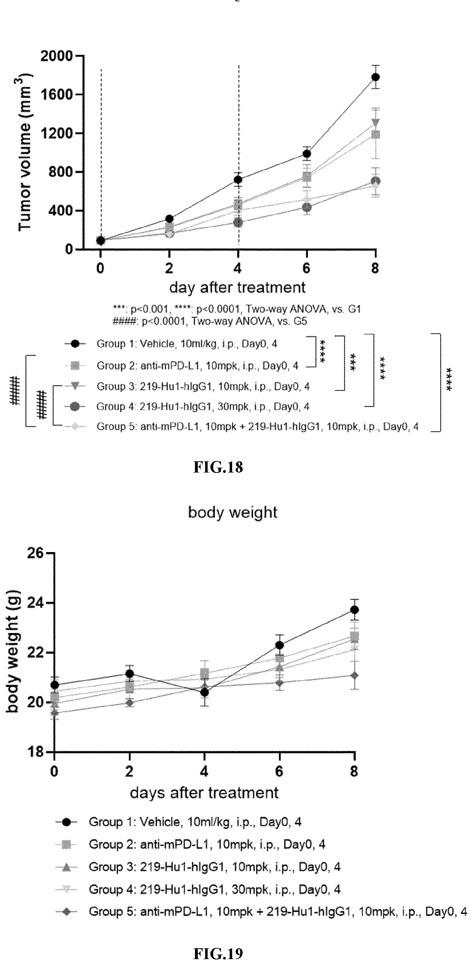

FIG. 18 shows the curves of tumour volume change in each treatment group in the hTNFR2 KI-CT26.1C03 colon cancer model.
FIG. 19 shows the curves of animal weight change in each treatment group in the hTNFR2 KI-CT26.1C03 colon cancer model. This figure shows the changes in absolute body weight of mice after dosing of different doses of 219-Hu1-hIgG1 antibodies or in combination with an anti-mPD-L1 antibody.

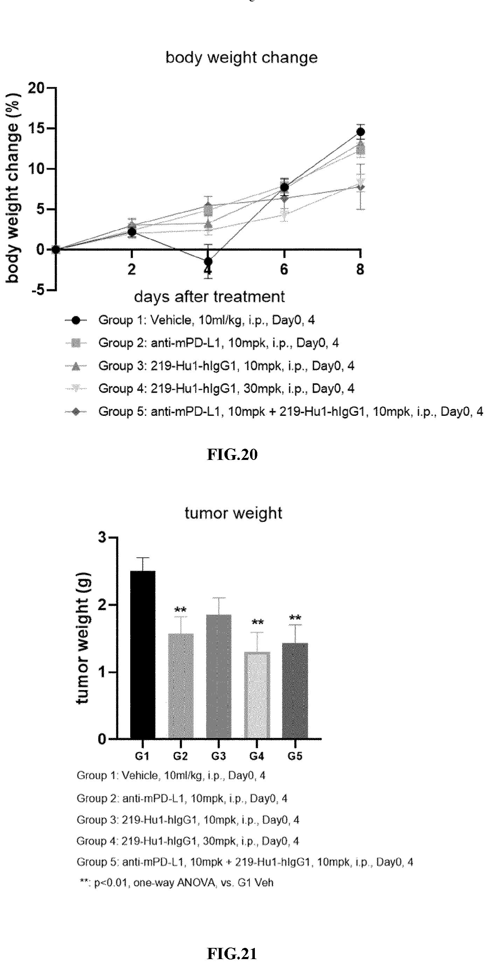

FIG. 20 shows the curves of animal weight change rate of each treatment group in the hTNFR2 KI-CT26.1C03 colon cancer model. This figure shows the change rate of body weight of mice after dosing of different doses of 219-Hu1-hIgG 1 antibodies or in combination with an anti-mPD-L1 antibody.
FIG. 21 shows the changes in tumour weight of each treatment group at the experimental end point in the hTNFR2 KI-CT26.1C03 colon cancer model. This figure shows the changes in weight of tumours isolated and weighed at the experimental end point after treatment with different doses of 219-Hu1-hIgG1 antibodies or in combination with an anti-mPD-L1 antibody.

### Detailed Description of Embodiments

The present invention is further described below in conjunction with specific examples. The advantages and features of the present invention will be more clearly described. If no specific conditions are indicated in the examples, conventional conditions or the conditions suggested by the manufacturer shall be followed. Any reagents or instruments used, unless the manufactures stated, are conventional products that are commercially available.

The examples of the present invention are only exemplary and do not limit the scope of the present invention in any way. It should be understood by those skilled in the art that the details and forms of the technical solutions of the present invention could be modified or substituted without departing from the spirit and scope of the present invention, but these modifications and substitutions all fall within the scope of protection of the present invention.

### Example 1 Expression and purification of a humanized monoclonal antibody against TNFR2

### 1.1 Humanization of an anti-TNFR2 antibody

The method of "transplantation of CDRs" was used to humanize an antibody, specifically, a human antibody with the highest homology was selected based on sequences to provide antibody framework regions (FRs), into which complementarity determining regions (CDRs) of an antigen-binding fragment in a target antibody based on the Kabat nomenclature were transplanted to form a humanized antibody variable region sequence in order of FR1-CDR1-FR2-CDR2-FR3-CDR3-FR4. Secondly, in order to effectively maintain the activity and affinity of an antibody, based on the antibody structure modelling (MOE software), sites for potential reverse mutation were selected, and the selection criteria were: 1) amino acid residues with antibody framework region located at the VH-VL interface, close to CDRs or directly interacting with CDRs were selected for reverse mutation; such amino acid residues mostly were important for maintaining the conformation of the CDR regions; 2) amino acids embedded in the protein were selected as far as possible for reverse mutation, considering the immunogenicity; 3) mutations with reduced molecular energy were preferred, considering the stability and expression level of an antibody. By testing a humanized antibody with different mutations for its affinity to human TNFR2 and for its binding to a cell expressing TNFR2 on the surface, a humanized antibody with the affinity, antibody characterization and activity function comparable to or better than a murine antibody was screened out.

The complementarity determining regions (CDRs) of an antigen-binding fragment in a TNFR2 antibody based on the Kabat nomenclature are detailed in Table 1. Table 2 shows the VH and VL sequences of a humanized antibody molecule. Table 3 shows the pairing of VH and VL sequences of a humanized antibody against TNFR2.

**Table 1. KABAT analysis results of CDRs of a candidate antibody (humanized antibody CDRs and murine antibody CDRs)**

| **TNFR2 antibody: CDR regions of a heavy chain according to the KABAT analysis** | | | | | | | | |
|---|---|---|---|---|---|---|---|---|
| **Antibody ID** | **SEQ ID NO.** | **CDR1-VH** | **SEQ ID NO.** | | **CDR2-VH** | | **SEQ ID NO.** | **CDR3-VH** |
| **#001** | 1 | TYDLS | 2 | | YINNGGISTYYSDTVKG | | 3 | GPFYGSANYFDY |
| **#219** | 7 | NYWMN | 8 | | MIHPSDTETRLNQNFKD | | 9 | GEGLGAARSVSMD Y |
| **#224** | 13 | DIYMH | 14 | | RIDPATGNTKHDPKFQD | | 15 | SPYGDFGAMDY |

| **TNFR2 antibody: CDR regions of a light chain according to the KABAT analysis** | | | | | | | | |
|---|---|---|---|---|---|---|---|---|
| **Antibody ID** | **SEQ ID NO.** | **CDR1-VL** | | **SEQ ID NO.** | | **CDR2-VL** | **SEQ ID No.** | **CDR3-VL** |
| **4001** | 4 | RTSESIYSNLP | | 5 | | DATKLAE | 6 | QHFWVTPWT |
| **#219** | 10 | RASQDISNYLN | | 11 | | YTAILHS | 12 | QQGNTLPWT |
| **#224** | 16 | TASSSVSSNYL H | | 17 | | STSNLPS | 18 | HQYHRSPWT |

**Table 2. VH and VL sequences of a humanized antibody against TNFR2**

| **VH** | | |
|---|---|---|
| **No.** | **SEQ ID NO.** | **Amino acid sequences** |
| VH1 | 19 | EVQLVESGGGLVQPGGSLRLSCAASGFAFSTYDLSWVRQAPGKGLEWVAYINNGG ISTYYSDTVKGRFTISRDNAKNTLYLQMNSLRAEDTAVYYCVRGPFYGSANYFDY WGQGTLVTVSS |
| VH2 | 20 | EVQLVESGGGLVQPGGSLRLSCAASGFAFSTYDLSWVRQAPGKGLEWVAYINNGG ISTYYSDTVKGRFTISRDNAKNSLYLQMNSLRAEDTAVYYCVRGPFYGSANYFDY |
| | | WGQGTLVTVSS |
| VH3 | 21 | QVQLVQSGAEVKKPGASVKVSCKASGYSFTNYWMNWVRQAPGQRLEWMGMIH PSDTETRLNQNFKDRVTITRDTSASTAYMELSSLRSEDTAVYYCARGEGLGAARSV SMDYWGQGTLVTVSS |
| VH4 | 22 | QVQLVQSGAEVKKPGSSVKVSCKASGYSFTNYWMNWVRQAPGQGLEWMGMIHP SDTETRLNQNFKDKATITADKSTSTAYMELSSLRSEDTAVYYCARGEGLGAARSVS MDYWGQGTLVTVSS |
| VH5 | 23 | QVQLVQSGAEVKKPGSSVKVSCKASGYSFTNYWMNWVRQAPGQGLEWMGMIHP SDTETRLNQNFKDRVTITADKSTSTAYMELSSLRSEDTAVYYCARGEGLGAARSVS MDYWGQGTLVTVSS |
| VH6 | 24 | QVQLVQSGAEVKKPGSSVKVSCKASGYSFTNYWMNWVKQAPGQGLEWIGMIHP SDTETRLNQNFKDKATLTVDKSTSTAYMELSSLRSEDTAVYYCARGEGLGAARSVS MDYWGQGTLVTVSS |
| VH7 | 25 | QVQLVQSGAEVKKPGSSVKVSCKASGYSFTNYWMNWVRQAPGQGLEWMGMIHP SDTETRLNQNFKDRVTITVDKSTSTAYMELSSLRSEDTAVYYCARGEGLGAARSVS MDYWGQGTLVTVSS |
| VH8 | 26 | QVQLVQSGAEVKKPGASVKVSCKASGFNNKDIYMHWVRQAPGQGLEWIGRIDPA TGNTKHDPKFQDRVTMTSDTSTNTVYMELSSLRSEDTAVYYCARSPYGDFGAMD YWGQGTLVTVSS |
| VH9 | 27 | QVQLVQSGSELKKPGASVKVSCKASGFNNKDIYMHWVRQAPGQGLEWMGRIDP ATGNTKHDPKFQDKAVLSSDTSVSTAYLQISSLKAEDTAVYYCARSPYGDFGAM DYWGQGTLVTVSS |

| **VL** | | |
|---|---|---|
| **No.** | **SEQ ID NO.** | **Amino acid sequences** |
| VL1 | 28 | DIQMTQSPSSLSASVGDRVTITCRTSESIYSNLPWYQQKPGKAPKLLLYDATKLAEG VPSRFSGSGSGTDYTLTISSLQPEDFATYYCQHFWVTPWTFGQGTKVEIK |
| VL2 | 29 | DIQMTQSPSSLSASVGDRVTITCRTSESIYSNLPWYQQKPGKSPKLLIYDATKLAEG VPSRFSGSGSGTDFTLTISSLQPEDFATYYCQHFWVTPWTFGQGTKVEIK |
| VL3 | 30 | DIQMTQSPSSLSASVGDRVTITCRTSESIYSNLPWYQQKPGKAPKLLIYDATKLAEG VPSRFSGSGSGTDYTLTISSLQPEDFATYYCQHFWVTPWTFGQGTKVEIK |
| VL4 | 31 | DIQMTQSPSSLSASVGDRVTITCRTSESIYSNLPWYQQKPGKSPKLLVYDATKLAEG VPSRFSGSGSGTDYTLTISSLQPEDFATYYCQHFWVTPWTFGQGTKVEIK |
| VL5 | 32 | DIQMTQSPSSLSASVGDRVTITCRASQDISNYLNWYQQKPDGTVKLLIYYTAILHSG VPSRFSGSGSGTDYSLTISSLQPEDFATYFCQQGNTLPWTFGQGTKVEIK |
| VL6 | 33 | DIQMTQSPSSLSASVGDRVTITCTASSSVSSNYLHWYQQKPGKAPKLWIYSTSNLP SGVPSRFSGSGSGTDFTLTISSLQPEDFATYYCHQYHRSPWTFGQGTKVEIK |
| VL7 | 34 | DIQMTQSPSSLSASVGDRVTITCTASSSVSSNYLHWYQQKPGSSPKLWIYSTSNLPS GVPSRFSGSGSGTDFTLTISSLQPEDFATYYCHQYHRSPWTFGQGTKVEIK |
| VL8 | 35 | DIQMTQSPSSLSASVGDRVTITCTASSSVSSNYLHWYQQKPGKAPKLLIYSTSNLPS GVPSRFSGSGSGTDFTLTISSLQAEDAATYYCHQYHRSPWTFGQGTKVEIK |
| VL9 | 36 | DIQMTQSPSSLSASVGDRVTITCTASSSVSSNYLHWYQQKPGKAPKLLIYSTSNLPS GVPSRFSGSGSGTDYSLTISSLQPEDFATYYCHQYHRSPWTFGQGTKVEIK |
| VL10 | 37 | DIQMTQSPSSLSASVGDRVTITCTASSSVSSNYLHWYQQKPGKAPKLLIYSTSNLPS GVPSRFSGSGSGTDFTLTISSLQPEDFATYYCHQYHRSPWTFGQGTKVEIK |

**Table 3. Pairing of VH and VL sequences of a humanized antibody against TNFR2**

| **Antibody ID** | **VH** | **VL** |
|---|---|---|
| 001-Hu1 | VH1 (SEQ ID NO.19) | VL1 (SEQ ID NO.28) |
| 001-Hu2 | VH2 (SEQ ID NO.20) | VL2 (SEQ ID NO.29) |
| 001-Hu3 | VH1 (SEQ ID NO.19) | VL3 (SEQ ID NO.30) |
| 001-Hu4 | VH2 (SEQ ID NO.20) | VL4 (SEQ ID NO.31) |
| 001-Hu5 | VH1 (SEQ ID NO.19) | VL4 (SEQ ID NO.31) |
| 219-Hu1 | VH3 (SEQ ID NO.21) | VL5 (SEQ ID NO.32) |
| 219-Hu2 | VH4 (SEQ ID NO.22) | VL5 (SEQ ID NO.32) |
| 219-Hu3 | VH5 (SEQ ID NO.23) | VL5 (SEQ ID NO.32) |
| 219-Hu4 | VH6 (SEQ ID NO.24) | VL5 (SEQ ID NO.32) |
| 219-Hu5 | VH7 (SEQ ID NO.25) | VL5 (SEQ ID NO.32) |
| 224- Hu1 | VH8 (SEQ ID NO.26) | VL6 (SEQ ID NO.33) |
| 224- Hu2 | VH9 (SEQ ID NO.27) | VL7 (SEQ ID NO.34) |
| 224- Hu3 | VH9 (SEQ ID NO.27) | VL8 (SEQ ID NO.35) |
| 224- Hu4 | VH9 (SEQ ID NO.27) | VL6 (SEQ ID NO.33) |
| 224- Hu5 | VH9 (SEQ ID NO.27) | VL9 (SEQ ID NO.36) |
| 224- Hu6 | VH8 (SEQ ID NO.26) | VL7 (SEQ ID NO.34) |
| 224- Hu7 | VH9 (SEQ ID NO.27) | VL10 (SEQ ID NO.37) |

### 1.2 Expression of a humanized antibody against TNFR2

The day before transfection, ExpiCHO-S cells were counted, and inoculated into a freshly preheated ExpiCHO expressing medium (Invitrogen, A291002) at a density of 2.5 - 4 × 10⁶ cells/mL for overnight culture. On the day of transfection, the ExpiCHO-S cell suspension cultured overnight was taken for counting; as a result, the cell concentration was about 7 - 10 × 10⁶ cells/mL, and the viability of cells for transfection was greater than 95%. According to the number of transfected cells, a required number of cells were taken, diluted with the ExpiCHO expressing medium to a final concentration of a density of 6 × 10⁶ cells/mL, and then placed in a shaker with 8% CO₂, at 37°C and 100 rpm for later use. Transfection preparation: a plasmid was diluted in an OptiPRO^{™} SFM medium (Invitrogen, 12309019), and the mixture was mixed well by gently shaking a centrifuge tube. A well-mixed ExpiFectamine^{™} CHO reagent (Invitrogen, A29129) was added to the plasmid diluent, and the mixture was mixed well by gently shaking the centrifuge tube, and then left to stand at room temperature for 2 minutes. The above-mentioned plasmid/ExpiFectamine^{™} CHO reagent complex was slowly added dropwise into a cell suspension to be transfected, during which a shake flask was shaken. After transfection, the transfected cells were cultured in a shaker with 8% CO₂, at a temperature of 37°C and a speed of 100 rpm. On Day 1 after transfection, the transfected cells were supplemented with 0.6% ExpiFectamine^{™} CHO Enhancer (Invitrogen, A29129) and 16% ExpiCHO^{™} Feed (Invitrogen, A29129), during which the shake flask was gently shaken, and then the cells were transferred to a shaker with 5% CO₂, at a temperature of 32°C and a speed of 100 rpm for culture. On Day 5 after transfection, the transfected cells were supplemented with 16% ExpiCHO^{™} Feed, during which the shake flask was gently shaken. On Day 12 after transfection, a cell supernatant was collected and centrifuged at 4000 g for 10 minutes, and then the supernatant was pipetted for further purification of the antibody.

### 1.3 Purification of a humanized antibody against TNFR2

The cell culture supernatant collected by high-speed centrifugation was filtered with a 0.45 + 0.22 µM filter membrane, and the filtrate was subjected to affinity chromatography using Akta Avant 150 (Cytiva) for the first step of purification. The chromatographic medium was Protein A filler Mabselect PrismA (Cytiva, Cat# 17549803) that interacted with Fc, and the equilibration buffer was PBS (2.5 g/L Na₂HPO₄ 12H₂O, 0.408 g/L NaH₂PO₄, and 8.76 g/L NaCl, pH 7.2). After the column was equilibrated with 4 column volumes of the equilibration buffer, the cell supernatant was loaded onto the column for binding, with the flow rate controlled at a point where the retention time of the sample on the column is ≥ 5 min. After sample loading, the column was washed with PBS (pH 7.2) until the ultraviolet absorption value at A280 dropped to the baseline. Then the column was eluted with 2 column volumes of 20 mM PB+1 M NaCl (3.752 g/L Na₂HPO4·12H₂O, 1.314 g/L NaH₂PO₄, and 58.44 g/L NaCl, pH 6.2). Subsequently, the column was washed with PBS (pH 7.2) until the ultraviolet absorption value at A280 and the conductivity reached the baseline. Finally, the chromatographic column was washed with an elution buffer of 20 mM citric acid (2.184 g/L citric acid, and 1.086 g/L sodium citrate, pH 3.4), and the elution peak was collected according to the ultraviolet absorption peak at A280. The collected elution sample was neutralized with 1 M Tris (121.14 g/L Tris) and detected to be neutral using a pH meter, Seven2Go (METTLER TOLEDO). The collected antibody was identified to be more than 95% pure by SEC-HPLC and used for follow-up studies.

### Example 2 Specific binding of a humanized antibody against TNFR2 to human and cynomolgus monkey TNFR2 proteins detected by ELISA

0.5 µg/ml human TNFR2 (Human TNFR2-His, Sino 10417-H08H) or cynomolgus monkey TNFR2 (Cynomolgus TNFR2-His, Sino 90102-C08H) was pre-coated with 100 µl/well of the antibody in an ELISA plate. The test humanized antibody against TNFR2 (as described in Example 1, the antibody was comprised of variable regions linked to IgG-Fc; the WT chimeric antibody also adopted the IgG-Fc structure) was subjected to 3.33-fold gradient dilution, loaded with 100 µl/well, and incubated with shaking at room temperature for 1.5 hours. The plate was washed, and then a working solution of murine anti-human IgG Fc-HRP (diluted at 1 : 10000) was added, loaded with 100 µl/well, and incubated with shaking at room temperature for 1.0 hour. The plate was washed again, and then the substrate TMB of HRP was added to develop colour. A stop solution was added to stop the reaction, and then a microplate reader (MD I3) was used to read the absorbance value. The binding curve of an antibody was drawn with antibody concentration as the abscissa and corresponding OD value as the ordinate, and fitted with four parameters (GraphPad Prism9) to calculate the EC50 value. The smaller the EC50 value, the stronger the binding ability of an antibody to human/cynomolgus monkey TNFR2. The binding effect of a humanized antibody was normalized to its corresponding human-murine chimeric antibody WT (see SEQ ID NOs: 38-43 for the sequence). The higher the percentage value, the better the binding effect of the humanized antibody. The results of binding of an anti-TNFR2 antibody to a human TNFR2 protein are shown in FIG. 1 and Table 4, and the results of binding of an anti-TNFR2 antibody to cynomolgus monkey TNFR2 are shown in FIG. 2 and Table 4. The data show that all the test antibodies can specifically bind to human or monkey TNFR2 proteins, and the binding ability of a humanized antibody is substantially the same as that of a human-murine chimeric antibody.

**Table 4. ELISA results of the specific binding of an anti-TNFR2 antibody to a human or cynomolgus monkey TNFR2 protein**

| **Sample No.** | **Binding to a human TNFR2 protein** | | **Binding to a cynomolgus monkey TNFR2 protein** | |
|---|---|---|---|---|
| | **EC50** | **% Relative activity** | **EC50** | **% Relative activity** |
| | **(ng/ml)** | **(WT antibody/test antibody)** | **(ng/ml)** | **(WT antibody/test antibody)** |
| 001-Hu1 | 9.86 | 103.9 | 9.62 | 108.2 |
| 001-Hu2 | 9.563 | 102.3 | 9.14 | 107.1 |
| 001-Hu3 | 10.15 | 100.9 | 9.9 | 105.1 |
| 001-Hu4 | 8.941 | 109.4 | 9.27 | 105.7 |
| 001-Hu5 | 10.23 | 100.1 | 988 | 105.4 |
| 219-Hu1 | 21.34 | 83.1 | 14.77 | 77.5 |
| 219-Hu2 | 18.48 | 95.9 | 11.27 | 101.6 |
| 219-Hu3 | 21.01 | 84.4 | 12.67 | 90.4 |
| 219-Hu4 | 16.75 | 99.7 | 12.25 | 93.1 |
| 219-Hu5 | 17.31 | 96.5 | 12.21 | 93.4 |
| 224-Hu1 | 10.44 | 142.8 | 9.981 | 104.8 |
| 224-Hu2 | 10.84 | 137.5 | 9.619 | 108.7 |
| 224-Hu3 | 12.18 | 122.4 | 9.686 | 108 |
| 224-Hu4 | 10.68 | 107.8 | 9.561 | 107.9 |
| 224-Hu5 | 11.58 | 99.4 | 9.686 | 106.5 |
| 224-Hu6 | 11.05 | 104.2 | 9.97 | 103.5 |
| 224-Hu7 | 9.604 | 109.6 | 8.393 | 116.1 |

| | | | | |
|---|---|---|---|---|
| 001-WT VH amino acid sequence (SEQ ID NO.38): EVQLQESGGGLVQPGGSLNLSCAASGFAFSTYDLSWVRQTPEKRLEWVAYINNGGISTYYSDTVKG RFTISRDNAKNTLYLQMSSLKSEDTAMYYCVRGPFYGSANYFDYWGQGTTVTVSS 001-WT VL amino acid sequence (SEQ ID NO.39): EIVMTQSPASLSLSVGETVTITCRTSESIYSNLPWYQQKQGKSPQLLVYDATKLAEGVPSRFSGSESG TQYSLKINSLQSEDFGTYYCQHFWVTPWTFGGGTKLEIK 219-WT VH amino acid sequence (SEQ ID NO.40): EVQLQESGAEL VRPGASVKLSCKASGYSFTTTYWNINW VKQRPGQGLEWIGMIHPSDTETRLNQNFK DKATLTVDKSSSTSYMQLSSPTSEDSAVYYCARGEGLGAARSVSMDYWGQGTTVTVSS 219-WT VL amino acid sequence (SEQ ID NO.41): DILMTQSPSSLSASLGDRVTISCRASQDISNYLNWYQQKPDGTVKVLIYYTAILHSGVPSRFSGSGSG TDYSLTISNLEQEDIATYFCQQGNTLPWTFGGGTKLEIK 224-WT VH amino acid sequence (SEQ ID NO.42): EVQLQESGPEIVKPGASVKLSCTASGFNNKDIYMHWVKQRPEQGLEWIGRIDPATGNTKHDPKFQD KATLSSDTSSNTAYLQFSSLTSEDTAVYYCAHSPYGDFGAMDYWGQGTTVTVSS 224-WT VL amino acid sequence (SEQ ID NO.43): DIQMTQSPAIMSASLGERVTMTCTASSSVSSNYLHWYQQKPGSSPKLWIYSTSNLPSGVPARFSGSGS GTSYSLTISSMEAEDAATYYCHQYHRSPWTFGGGTKLEIK | | | | |

### Example 3 Affinity of a humanized antibody against TNFR2 for human and cynomolgus monkey TNFR2 proteins detected by BIAcore

Biacore was used to detect the specific binding of a test anti-TNFR2 antibody to human and cynomolgus monkey TNFR2 proteins. In this experiment, a Protein A chip was used; the time required for the chip to capture the diluted antibody was determined by manual run, so that the Rmax of the saturated binding to an antigen was 50 RU. Both human (Human TNFR2-His, Sino 10417-H08H) and cynomolgus monkey (Cynomolgus TNFR2-His, Sino 90102-C08H) TNFR2 proteins were subjected to gradient dilution to 32, 16, 8, 4, and 2 nM. The affinity of an antibody for an antigen was determined by using multi-cycle dynamics. In each cycle, an antibody was injected and then a TNFR2 protein at a gradient concentration was injected, so that the antigen and the antibody underwent binding and dissociation processes. After each cycle, the Protein A chip was regenerated with Glycine pH 1.5 (removing the protein on the chip). The affinity KD of an antibody and an antigen was fitted with BIAcore T200 analysis software. The results in Table 5 show that all the test anti-TNFR2 antibodies specifically bind to a human or cynomolgus monkey TNFR2 protein with a high affinity level.

**Table 5. BIAcore results of the specific binding of an anti-TNFR2 antibody to a human or cynomolgus monkey TNFR2 protein**

| **Antibody No.** | **Antigen** | **KD (M)** | **ka (1/Ms)** | **kd (1/s)** | **Antigen** | **KD (M)** | **ka (1/Ms)** | **kd (1/s)** |
|---|---|---|---|---|---|---|---|---|
| 001-WT | Human TNFR2 | 2.35E -10 | 5.12E+05 | 1.21E-04 | Cynomolgus monkey TNFR2 | 2.52E-10 | 6.14E+05 | 1.54E-04 |
| 001-Hu1 | | 3.71E -10 | 2.91E+05 | 1.08E-04 | | 4.70E-10 | 3.40E+05 | 1.60E-04 |
| 001-Hu2 | | 4.96E -10 | 2.15E+05 | 1.07E-04 | | 5.99E-10 | 2.73E+05 | 1.64E-04 |
| 001-Hu3 | | 3.75E -10 | 2.65E+05 | 9.91E-05 | | 4.30E-10 | 3.61E+05 | 1.55E-04 |
| 001-Hu4 | | 4.17E -10 | 2.70E+05 | 1.13E-04 | | 4.73E-10 | 3.54E+05 | 1.67E-04 |
| 001-Hu5 | | 4.16E -10 | 2.77E+05 | 1.15E-04 | | 4.75E-10 | 3.69E+05 | 1.75E-04 |
| 219-WT | | 5.54E -10 | 3.46E+05 | 1.92E-04 | | 1.14E-09 | 4.92E+05 | 5.62E-04 |
| 219-Hu1 | | 3.54E -10 | 4.33E+05 | 1.53E-04 | | 5.32E-10 | 6.23E+05 | 3.32E-04 |
| 219-Hu2 | | 3.40E -10 | 4.43E+05 | 1.51E-04 | | 6.81E-10 | 6.05E+05 | 4.12E-04 |
| 219-Hu3 | | 3.62E-10 | 4.03E+05 | 1.46E-04 | | 7.23E-10 | 5.52E+05 | 3.99E-04 |
| 219-Hu4 | | 3.18E -10 | 4.84E+05 | 1.54E-04 | | 5.76E-10 | 6.75E+05 | 3.89E-04 |
| 219-Hu5 | | 3.09E -10 | 4.45E+05 | 1.37E-04 | | 6.26E-10 | 6.08E+05 | 3.81E-04 |
| 224-WT | | 2.33E -09 | 1.74E+05 | 4.05E-04 | | 8.97E-10 | 4.56E+05 | 4.09E-04 |
| 224-Hu1 | | 6.57E -09 | 1.17E+05 | 7.66E-04 | | 3.95E-09 | 2.41E+05 | 9.53E-04 |
| 224-Hu2 | | 4.31E -09 | 1.59E+05 | 6.86E-04 | | 2.95E-09 | 2.83E+05 | 8.35E-04 |
| 224-Hu3 | | 4.10E -09 | 1.77E+05 | 7.26E-04 | | 2.97E-09 | 2.85E+05 | 8.47E-04 |
| 224-Hu4 | | 5.59E -09 | 1.29E+05 | 7.20E-04 | | 3.17E-09 | 2.78E+05 | 8.81E-04 |
| 224-Hu5 | | 4.28E -09 | 1.59E+05 | 6.82E-04 | | 3.31E-09 | 2.50E+05 | 8.28E-04 |
| 224-Hu6 | | 4.08E -09 | 1.71E+05 | 6.97E-04 | | 2.63E-09 | 3.23E+05 | 8.48E-04 |
| 224-Hu7 | | 5.64E -09 | 1.45E+05 | 8.15E-04 | | 3.12E-09 | 3.18E+05 | 9.91E-04 |

### Example 4 Binding of a TNFR2 antibody to human TNFR2 on the surface of CHO-TNFR2 cells detected by FACS

CHO-K1 stable cells transfected with a high-expression plasmid of human TNFR2 (named CHO-TNFR2) were taken. The transfected full-length plasmid of human TNFR2 was purchased from Sino Biological (Cat# HG10417-UT, NCBI Ref Seq: NM_001066.2). The experiment was conducted at a cell density within 80%. The cell medium was discarded; the cells were rinsed with PBS, and digested with 1 ml trypsin for 2 minutes; the digestion was stopped with a Ham's F12 complete medium containing 10% FBS, and then a cell suspension was made. The cell suspension was counted, and then an appropriate amount of cell suspension was centrifuged at 350×g; the supernatant was discarded; a corresponding volume of blocking solution (10% FBS + PBS) at a density of 1 × 10⁷ cells/ml was added to resuspend the cells, and the mixture was incubated at 4°C for 30 minutes. After the incubation, the mixture was centrifuged at 350×g to remove the supernatant, and the cells were resuspended in a staining buffer (2% FBS + PBS) to a density of 2 × 10⁶ cells/ml, and spread onto a 96-well plate, with 50 µl of cell suspension added to each well for later use. The antibody was diluted with PBS for 10-fold gradient dilution from the highest concentration of 20 µg/ml (twice the concentration); the diluted antibody was added to the well containing 50 µl of cell suspension, and placed on a microplate shaker for shaking at 500 rpm for 1 minute, so that the antibody was fully mixed with the cells; the mixture was incubated at 4°C for 1 h. After the incubation, the cells were washed twice with the staining buffer, with 100 µl per well, and centrifuged at 350×g for 5 minutes, and the supernatant was discarded. The PE goat anti-Human IgG Fc antibody (ebioscience, Cat# 12-4998-82) was diluted 250-fold with the staining buffer, with a volume of 100 µl per well added to the washed cell wells; the mixture was mixed evenly, and stained at 4°C for 30 minutes. After the staining, similarly, the cells were washed twice with the staining buffer; finally, the cells were resuspended in 200 µl of staining buffer; signals were detected by a flow cytometer (Thermo Attune NxT). The stronger the signal, the stronger the binding ability of an antibody to TNFR2. The binding curve of an antibody was drawn with antibody concentration as the abscissa and corresponding mean fluorescence intensity (MFI) fold as the ordinate, and fitted with four parameters (GraphPad Prism9) to calculate the EC50 value. The smaller the EC50 value, the stronger the binding ability of an antibody to a CHO-TNFR2 cell. The binding effect of a humanized antibody was normalized to its corresponding human-murine chimeric WT antibody. The higher the percentage value, the better the binding effect of the humanized antibody. As shown in FIG. 3 and Table 6, all the test anti-TNFR2 antibodies can bind to human TNFR2 on the surface of CHO-TNFR2 cells, and the binding ability of a humanized antibody is substantially the same as that of a human-murine chimeric WT antibody.

**Table 6. Flow cytometry results of the specific binding of an anti-TNFR2 antibody to human TNFR2 on the surface of CHO-TNFR2 cells**

| **Sample No.** | **Binding to a CHO-TNFR2 cell** | | | |
|---|---|---|---|---|
| | **EC50** | **% Relative activity** | **AUC** | **% Relative activity** |
| | **(ng/ml)** | **(WT antibody/test antibody)** | **NA** | **(Test antibody/WT antibody)** |
| 001-WT | 0.035 | 100 | 490.4 | 100 |
| 001-Hu1 | 0.045 | 78.03 | 458 | 93.39 |
| 001-Hu2 | 0.073 | 48 | 432.6 | 88.21 |
| 001-Hu3 | 0.035 | 100.72 | 430.6 | 87.81 |
| 001-Hu4 | 0.027 | 129.01 | 452.3 | 92.23 |
| 001-Hu5 | 0.029 | 122.82 | 430.7 | 87.83 |
| 219-WT | 0.026 | 100 | 515.6 | 100 |
| 219-Hu1 | 0.026 | 100.15 | 564.8 | 109.54 |
| 219-Hu2 | 0.027 | 97.09 | 533.5 | 103.47 |
| 219-Hu3 | 0.018 | 145.02 | 497.1 | 96.41 |
| 219-Hu4 | 0.018 | 141.94 | 493.1 | 95.64 |
| 219-Hu5 | 0.018 | 146.82 | 481 | 93.29 |
| 224-WT | 0.04 | 100 | 667.4 | 100 |
| 224-Hu1 | 0.04 | 94.54 | 642 | 96.19 |
| 224-Hu2 | 0.03 | 119.14 | 671.6 | 100.63 |
| 224-Hu3 | 0.03 | 116.13 | 674.6 | 101.08 |
| 224-Hu4 | 0.03 | 130.53 | 621.1 | 93.06 |
| 224-Hu5 | 0.04 | 111.75 | 593.1 | 88.87 |
| 224-Hu6 | 0.03 | 138.8 | 603.1 | 90.37 |
| 224-Hu7 | 0.03 | 140.34 | 607.7 | 91.05 |

### Example 5 Binding experiment of an antibody to TNFR2 on the surface of human regulatory T cells (Treg cells) detected by FACS

Human Treg cells were isolated from human PBMCs by using a sorting kit (Stemcell, Cat# 18063), stimulated and expanded *in vitro* by Dynabeads Human Treg Expander (Gibco, Cat# 11129D) for 15 days, and then packaged and frozen for storage. The Treg cells isolated and expanded *in vitro* were recovered overnight, centrifuged at 300×g for 5 minutes the next day, and resuspended in DPBS to obtain a cell suspension; the cell suspension was counted. The cells required for the experiment were added to a centrifuge tube, centrifuged at 300×g for 5 min to remove the supernatant, adjusted to a density of 2 × 10⁶ cells/ml with a staining buffer, and spread onto a 96-well plate, with 50 µl per well. All the test antibodies and the control antibody anti-Hel isotype (diluted to 200 ng/ml with PBS, respectively, with a total amount of 100 µl) were taken. Each sample was added to each well, with 50 µl per well. The well plate added with the cell suspension and the antibody was placed on a microplate shaker for shaking at a speed of 500 rpm for 1 min, so that the cells were fully mixed with the antibody. After the full mixing, the well plate was placed in a 4°C freezer, and incubated for 60 min. After the incubation, 100 µl of staining buffer was added to each well; the mixture was centrifuged at 350×g for 5 min; the supernatant was discarded. An additional 200 µl of staining buffer was added to each well to resuspend the cells; the cell suspension was centrifuged at 350×g for 5 min; the supernatant was discarded. The staining buffer was added to the PE goat anti-Human IgG Fc at a ratio of 250 : 1 (staining buffer : fluorescently stained antibody) for preparation of a staining solution; the solution was mixed well, and then added to the cell wells, with 100 µl per well. The well plate was placed on a microplate shaker for shaking at a speed of 500 rpm for 1 min, so that the cells were fully mixed with the staining solution. After the full mixing, the well plate was placed in a 4°C freezer, and incubated for 30 min. The cells were washed twice; finally, 200 µl of PBS was added to each well to resuspend the cells; signals were detected by a flow cytometer (Thermo Attune NxT). FIG. 4 shows that all the humanized antibodies against TNFR2 can effectively bind to human Treg cells.

### Example 6 Binding experiment of an antibody to activated cynomolgus monkey PBMC cells detected by FACS

The frozen cynomolgus monkey PMBC cells were recovered, and then resuspended with a complete medium (RPMI1640-Glutamax+10% FBS+1×P/S+1×ITS+50 µMPM mercaptoethanol) to obtain a cell suspension; the cell suspension was counted. According to the counting results, the resuspended cells were adjusted to a density of 5 × 10⁶ cells/ml; 100 µl of cell suspension was spread in each well of a 96-well U-bottom plate. A T cell activating reagent, Immunocult T cell (Stemcell, 1 : 50) activator +rhIL-2 (R&D, 200 IU), at 20% concentration was formulated; the activating reagent at 20% concentration was added to cells, with 100 µl per well; the mixture was mixed well, then placed in an incubator with 5% CO₂ at 37°C, and incubated for three days. The activated cells were collected and counted; the cells were adjusted to a density of 4 × 10⁶ cells/ml with a staining buffer (a DPBS solution containing 2% FBS), and spread onto a 96-well V-bottom plate, with 50 µl per well. All the test antibodies and the anti-Hel control antibody (diluted to 0.4 µg/ml and 4 µg/ml with a staining buffer, respectively) were taken; each sample was added to each well, with 50 µl per well. The well plate added with the cell suspension and the antibody was placed on a microplate shaker for shaking at a speed of 500 rpm for 1 min, so that the cells were fully mixed with the antibody. After the full mixing, the well plate was placed in a 4°C freezer, and incubated for 30 min. After the incubation, 100 µl of staining buffer was added to each well; the mixture was centrifuged at 350×g for 5 min; the supernatant was discarded. An additional 200 µl of staining buffer was added to each well to resuspend the cells; the cell suspension was centrifuged at 350×g for 5 min; the supernatant was discarded. The antibodies Anti-Human CD8-FTIC (BD-555366, 1 µl/test) and PE goat anti-Human IgG Fc (Invitrogen-124998-82, 0.5 µl/test) were added. The well plate was placed on a microplate shaker for shaking at a speed of 500 rpm for 1 min, so that the cells were fully mixed with the staining solution. After the full mixing, the well plate was placed in a 4 °C freezer, and incubated for 30 min. The cells were washed twice; finally, 200 µl of staining buffer was added to each well to resuspend the cells; signals were detected by a flow cytometer (Thermo Attune NxT). FIG. 5 shows that the Anti-Hel negative isotype control antibody does not bind to cynomolgus monkey CD8 T cells, and all the test humanized antibodies against TNFR2 can effectively bind to CD8+ T cells in activated cynomolgus monkey PBMCs.

### Example 7 Blocking of binding of TNFα to TNFR2 by an anti-TNFR2 antibody detected by ELISA

1 µg/ml human TNFR2 (Novoprotein, Cat# C830) was pre-coated with 100 µl/well of the antibody in an ELISA plate. All the test TNFR2 antibodies were subjected to 2.5-fold gradient dilution from the highest concentration of 30 µg/ml; the diluted antibodies were mixed with 15 ng/ml human TNF**α**-Biotin (Aero Biosystem, Cat# TNA-H82E3) in equal volume, respectively; the resulting mixture was added to the ELISA plate at 100 µl/well, and incubated with shaking at room temperature for 2.0 hours. The plate was washed, and then a Streptavidin-HRP working solution (diluted at 1 : 10000) was added at 100 µl/well, and the mixture was incubated with shaking at room temperature for 40 minutes. The plate was washed again, and then the substrate TMB of HRP was added to develop colour. A stop solution was added to stop the reaction, and then a microplate reader (MD I3) was used to read the absorbance value. The inhibitory curve of an antibody was drawn with antibody concentration as the abscissa and corresponding OD value as the ordinate, and fitted with four parameters (GraphPad Prism9) to calculate the IC50 value. The smaller the IC50 value, the stronger the ability of an antibody to inhibit the binding of human TNFα to human TNFR2. The blocking effect of a humanized antibody was normalized to its corresponding human-murine chimeric antibody WT. The higher the percentage value, the better the inhibitory effect of the humanized antibody. The blocking curve of test antibodies is shown in FIG. 6, and the inhibitory activity is shown in Table 7. As can be seen from FIG. 6 and Table 7, all the test antibodies can significantly inhibit the binding of human TNFα to a human TNFR2 protein, and the inhibitory ability of a humanized antibody is substantially the same as that of a human-murine chimeric WT antibody.

**Table 7. Blocking of binding of TNFα to TNFR2 by test antibodies**

| **Sample No.** | **Blocking of binding of TNFα to a human TNFR2 protein** | | | |
|---|---|---|---|---|
| | **IC50** | **% Relative activity** | **AUC** | **% Relative activity** |
| | **(ng/ml)** | **(WT antibody/test antibody)** | **NA** | **(WT antibody/test antibody)** |
| 001-Hu1 | 0.573 | 94.7 | 7.635 | 98 |
| 001-Hu2 | 0.7804 | 82.8 | 8.099 | 97.1 |
| 001-Hu3 | 0.5968 | 105.3 | 7.872 | 99.4 |
| 001-Hu4 | 0.5903 | 105.1 | 7.857 | 100.6 |
| 001-Hu5 | 0.6559 | 96.5 | 8.024 | 98 |
| 219-Hu1 | 0.4896 | 127.9 | 7.918 | 105.2 |
| 219-Hu2 | 0.4719 | 119.6 | 7.822 | 104.2 |
| 219-Hu3 | 0.5451 | 111.2 | 8.161 | 102.8 |
| 219-Hu4 | 0.6059 | 125.6 | 7.655 | 104.7 |
| 219-Hu5 | 0.5782 | 135.6 | 7.962 | 106.3 |
| 224-Hu1 | 0.4409 | 106.4 | 7.125 | 98.8 |
| 224-Hu2 | 0.4399 | 121.2 | 7.184 | 102 |
| 224-Hu3 | 0.4409 | 119.7 | 6.342 | 98.2 |
| 224-Hu4 | 0.4582 | 105.2 | 6.254 | 100.5 |
| 224-Hu5 | 0.5277 | 93.1 | 6.874 | 97.2 |
| 224-Hu6 | 0.4391 | 114.6 | 6.46 | 100 |
| 224-Hu7 | 0.5614 | 105.5 | 7.439 | 100 |

### Example 8 Blocking of binding of TNFR2 on CHO cells highly expressing human TNFR2 to TNFα by an anti-TNFR2 antibody

CHO stable cells highly expressing human TNFR2 were used for the experiment (the same as CHO-TNFR2 in Example 4). The CHO-TNFR2 cells were digested, washed twice with DPBS, then stained with Live/Dead (L/D) (20 minutes at room temperature), and plated at a density of 1 × 10⁵ cells/50 µl/well. A test anti-TNFR2 antibody was diluted to 6 µg/ml with a staining buffer as the starting concentration; the antibody was subjected to 3.33-fold gradient dilution, with a total of 7 concentration points. The diluted antibody was added to the plated cells at 50 µl/well, and the mixture was mixed well by gentle pipetting, and incubated at 4°C for 30 minutes. Then the diluted 100 ng/ml of human TNFα-biotin was added at 100 µl/well, and the mixture was mixed well by gentle pipetting, and then incubated at 4°C for 30 minutes. The mixture was washed twice with a staining buffer, and then 0.12 µg/ml PE-streptavidin (BioLegend, Cat# 405204) was added at 100 µl/well; the resulting mixture was incubated at 4°C for 30 minutes. The mixture was washed twice with a staining buffer, and then 150 µl of staining buffer was added to resuspend the cells; signals were detected by a flow cytometer (Thermo Attune NxT). The inhibitory curve of an antibody was drawn with antibody concentration as the abscissa and corresponding MFI value as the ordinate, and fitted with four parameters (GraphPad Prism9) to calculate the IC50 value. The smaller the IC50 value, the stronger the ability of an antibody to inhibit the binding of human TNFα to human TNFR2. The blocking effect of a humanized antibody was normalized to its corresponding human-murine chimeric WT antibody. The higher the percentage value, the better the inhibitory effect of the humanized antibody. The blocking curve of test antibodies is shown in FIG. 7, and the inhibitory activity is shown in Table 8. As can be seen from FIG. 7 and Table 8, all the test antibodies can significantly inhibit the binding of TNFα to TNFR2 on CHO-TNFR2 cells.

**Table 8. Blocking of binding of TNFα to TNFR2 on the surface of CHO cells by a TNFR2 antibody**

| **Sample No.** | **Blocking of binding of TNFα to a CHO-TNFR2 cell** | | | |
|---|---|---|---|---|
| | **IC50** | **% Relative activity** | **AUC** | **% Relative activity** |
| | **(ng/ml)** | **(WT antibody/test antibody)** | **NA** | **(WT antibody/test antibody)** |
| 001-Hu1 | 0.087 | 98.3 | 8839 | 97.4 |
| 001-Hu2 | 0.101 | 84.3 | 8995 | 95.8 |
| 001-Hu3 | 0.085 | 100 | 8809 | 97.8 |
| 001-Hu4 | 0.087 | 92.3 | 10174 | 93.7 |
| 001-Hu5 | 0.093 | 86.2 | 10241 | 93.1 |
| 219-Hu1 | 0.047 | 106.4 | 7316 | 102.2 |
| 219-Hu2 | 0.039 | 126.9 | 7336 | 101.9 |
| 219-Hu3 | 0.042 | 119.9 | 7590 | 98.5 |
| 219-Hu4 | 0.043 | 123.6 | 7320 | 108 |
| 219-Hu5 | 0.039 | 138.1 | 7260 | 108.9 |
| 224-Hu1 | 0.042 | 107.7 | 20307 | 99.2 |
| 224-Hu2 | 0.036 | 124.2 | 19249 | 104.6 |
| 224-Hu3 | 0.044 | 107 | 18665 | 95.6 |
| 224-Hu4 | 0.058 | 101.7 | 14188 | 97.3 |
| 224-Hu5 | 0.062 | 95 | 14625 | 94.4 |
| 224-Hu6 | 0.061 | 96 | 13882 | 99.4 |
| 224-Hu7 | 0.065 | 73 | 19578 | 91.1 |

### Example 9 Experiment of inhibition by an antibody on TNFα-induced degradation of IκBα in a human Treg cell detected by FACS

Human Treg cells were isolated from human PBMCs by using a sorting kit (Stemcell, Cat# 18063), stimulated and expanded *in vitro* by Dynabeads Human Treg Expander (Gibco, Cat# 11129D) for 15 days, and then packaged and frozen for storage. The Treg cells isolated and expanded *in vitro* were recovered overnight, centrifuged at 300×g for 5 minutes the next day, and resuspended with an AIM-V medium (brand: Gibco, Cat# 31035025); the cells were counted, adjusted to a density of 6 × 10⁶ cells/ml based on the cell counting results, and spread onto a 96-well plate, with 25 µl per well. All the test antibodies and the anti-Hel control antibody (diluted to 40% concentration with the AIM-V medium, 1 µg/ml, 10 µg/ml and 100 µg/ml respectively) were taken. Antibodies at each concentration were added to the corresponding cell well, with 25 µl per well. The mixture was mixed with a multi-channel pipette, and then pre-incubated for 30 min in an incubator with 5% CO₂ at 37°C. TNFαα (brand: Novoprotein, Cat# C008) was diluted with the AIM-V medium to 20 ng/ml (twice the working concentration); 50 µl of TNFα was added to the well requiring TNFα activation, and 50 µl of AIM-V medium was added to the control well requiring no activation, so that the final concentration of the antibody was 10%, and the final concentration of TNFαα was 10 ng/ml; the mixture was mixed well by pipetting; the culture plate was incubated for 10 min in an incubator at 37°C. After the incubation, the culture plate was taken out, into which 100 µl of pre-cooled DPBS was added, and the mixture was centrifuged at 4°C and 300g for 5 min. Then the cells were treated with 100 µl 1× live/dead violet (diluted 500-fold with pre-cooled DPBS), and reacted on ice for 20 min. 100 µl of staining buffer was added to each well; the mixture was centrifuged at 4°C and 300g for 5 min. 100 µl of 1×fix-perm buffer (brand: BD, Cat# 554714) was added to each well, mixed evenly, and fixed at 4°C for 30 min. After the fixation, 100 µl of 1×perm buffer was added to each well to terminate the fixation; the mixture was centrifuged at 400g for 5 min; the supernatant was discarded. Then 200 µl of 1 ×perm buffer was added to each well to wash the cells again. 100 µl of IκBα staining solution (diluted 100-fold with 1 ×perm buffer) was added to the experimental well, and 1 µl of Rat IgG2b **κ** isotype control antibody was added to the control well; the mixture was mixed evenly, and incubated at 4°C for 30 min. After the incubation, 100 µl of 1 ×perm buffer was added to each well to terminate the fixation; the mixture was centrifuged at 400g for 5 min; the supernatant was discarded. Then 200 µl of 1 ×perm buffer was added to each well to wash the cells again. Signals were detected by a flow cytometer (Thermo Attune NxT). Flow cytometry was used to analyse the percentage of IκBα ^{low} in viable cells or the MFI of IκBα in all viable cells. The lower the proportion of IκBαα ^{low}, the stronger the ability of an anti-TNFR2 antibody to inhibit the TNFα-induced degradation of IκBα. The results in FIG. 8 show that the negative control antibody Anti-Hel has no effect on the expression of IκBα, while all the test humanized antibodies against TNFR2 can inhibit the TNFα-induced degradation of IκBα.

### Example 10 Inhibition of proliferation of Treg cells induced by TNFα and IL-2 by a TNFR2 antibody

By measuring the proliferation of Treg cells induced by TNFα and IL-2 in the presence of a TNFR2 antibody, the inhibitory effect of this antibody on the proliferation of Treg cells induced by TNFα and IL-2 was determined (Zaragoza B et al., Nat Med. 2016 Jan; 22(1):16-7). The frozen Treg cells expanded and isolated *in vitro* were recovered overnight, centrifuged at 400×g for 5 minutes the next day, and resuspended with a medium to obtain a cell suspension; the cell suspension was counted. The Tregs were stained with CellTrace Violet cell proliferation kit (Thermo, Cat# C34557), with 1 µl of storage solution per 1 × 10⁷ cells used to prepare 1 ml of staining solution. Staining was performed at 37°C for 20 min; the reaction was neutralized with at least 5 times the volume of complete medium, left to stand for 5 min, and centrifuged at 400×g for 5 min. The cells were resuspended and washed once with a medium, and then the washed cells were centrifuged again to obtain the supernatant. The cells were plated at a density of 1 × 10⁵ cells per well of a 96-well plate, resuspended with a medium at a density of 1 × 10⁵ cells per 50 µl, and transferred to a 96-well plate. 50 µl of antibody to be tested was added to each well, with the final concentration of the antibody being 12.5 µg/ml; the mixture was incubated in an incubator for 30 minutes. Then 50 µl of medium containing IL-2 (with a final concentration of 300 ill) and TNFα (with a final concentration of 50 ng/ml) and 50 µl of medium containing anti-CD3/CD28 Dynabeads (Gibco, Cat# 11129D, with the ratio of beads to Treg cells of 1 : 20) were added to each well, with the final volume of each well reaching 200 µl. Two parallel wells were set for each condition; the above-mentioned well plates were mixed evenly, and then incubated in an incubator at 37°C for three days for FACS detection (Thermo Attune NxT). The inhibitory effect of the antibody on Treg proliferation induced by TNFα and IL-2 was determined by the proportion of Treg proliferation. Results in FIG. 9 show that all the humanized antibodies against TNFR2 can effectively inhibit the proliferation of Treg cells induced by TNFα and IL-2 compared with the control antibody anti-Hel, and there are significant differences.

### Example 11 Inhibition of Treg functions by an anti-TNFR2 antibody

By measuring the inhibitory function of Treg cells on responder T cells in the presence of an anti-TNFR2 antibody, the inhibitory effect of the antibody on the inhibitory function of Treg cells was determined. The effector cells, Tregs (CD4+CD25+FoxP3+ T cells) and Conventional T cells (Tcons) (CD4+CD25- T cells), required for the experiment were recovered overnight, centrifuged at 400×g for 5 minutes the next day, and resuspended with a complete medium (RPMI1640-Glutamax+10% FBS + 1×P/S+1×ITS+50 µMPM mercaptoethanol) to obtain a cell suspension; the cell suspension was counted. The Tcon cells were stained with CellTrace Violet cell proliferation kit (Thermo, Cat# C34557), with 1 µl of storage solution per 1 × 10⁷ cells used to prepare 1 ml of staining solution. Staining was performed at 37°C for 20 min; the reaction was neutralized with at least 5 times the volume of complete medium, left to stand for 5 min, and centrifuged at 400×g for 5 min. The cells were resuspended and washed once with a medium, and then the washed cells were centrifuged again to discard the supernatant. The cells were plated at a density of 1 × 10⁵ cells per well of a 96-well plate, resuspended with a medium at a density of 1 × 10⁵ cells per 50 µl, and transferred to a 50 mL centrifuge tube. The Treg cells were prepared at the same time, and the Treg cells and the Tcon cells were co-cultured at a ratio of 1 : 1. Corresponding anti-CD3/CD28 Dynabeads (Gibco, Cat# 11129D) were prepared at a ratio of 1/10 by the number of Tcon cells, and added to a medium containing Treg and Tcon at a volume of 50 µl per well. An antibody to be tested was added to each well, with the final concentration of the antibody being 12.5 µg/ml. Three parallel wells were set for each condition; the above-mentioned well plates were mixed evenly, and then incubated in an incubator at 37°C for 4 days for flow cytometry. The higher the proliferation proportion of Tcon, the stronger the ability of an antibody to inhibit Treg. The inhibitory results as shown in FIG. 10 indicate that all three test humanized antibodies against TNFR2 can significantly inhibit the inhibitory effect of Tcon cells by Tregs and promote the proliferation of Tcon, and there are statistical differences.

### Example 12 Antibody-dependent cell-mediated cytotoxicity (ADCC) mediated by an anti-TNFR2 antibody detected by FACS

PBMCs and the frozen Treg cells were cultured in a complete medium (RPMI1640-Glutamax+10% FBS+1×P/S+1×ITS+50 µMPM mercaptoethanol) one day in advance, in which 100 IU/ml of IL-2 was added to the PBMCs. On the day of the experiment, NK cells were isolated from PBMCs according to the requirements of the isolation kit (Stemcell, Cat# 17955), and resuspended with a complete medium (without IL-2) at a density of 0.8 × 10⁶ cells/ml. The target Treg cells or CHO-TNFR2 cells were labelled with a Cell Trace violet reagent; after the labelling, the cells were adjusted to a density of 0.4 × 10⁶ cells/ml. An antibody to be tested was subjected to gradient dilution with a complete medium to a concentration 4 times the final concentration for later use. According to the distribution map of the experimental plate, the target cells were seeded into a cell plate, with 50 µl per well, and then the diluted drug was spread onto a corresponding well, and co-incubated with the target cells at 37°C for 30 minutes. After the incubation, 100 µl of effector cell suspension was added to a well requiring effector cells, and 100 µl of medium was added to a well requiring no effector cells; the mixture was incubated at 37°C for 4 h. After the reaction, 1 µl of PI dye was added to each well for detection on an instrument. The higher the proportion of PI positive in target cells, the more significant the effect of ADCC. The results of ADCC experiment as shown in FIG. 11 indicate that the anti-Hel negative control antibody exhibits no killing effect of ADCC due to the lack of effect targets; all three test humanized antibodies show an ADCC activity on the target Treg cells or CHO-TNFR2 cells.

### Example 13 Detection of hTNFR2 and mTNFR2 expression in TNFR2 transgenic humanized mice (TNFR2 HuGEMM)

By using CRISPR/Cas9 technology and homologous recombination, the hTNFRSF1B-WPRE-PA expression cassette was knocked into the exon2 site of Tnfrsflb gene in a site-specific manner to obtain a heterozygous BALB/c mouse with the Tnfrsflb gene knocked into hTNFRSF1B-WPRE-PA in a site-specific manner; then a homozygous transgenic mouse (TNFR2 HuGEMM) expressing human Tnfrsflb (TNFR2) gene was obtained through breeding. The peripheral blood from a 6-8-week-old female BALB/c mouse (from Beijing Vital River Laboratory Animal Technology Co., Ltd.) and a TNFR2 HuGEMM mouse was treated with ACK (Gibco, Cat# A1049201) to remove red blood cells; the treated peripheral blood was subjected to immunophenotyping by FACS to detect the expression of mTNFR2 and hTNFR2 on CD4+ T cells, CD8+ T cells and CD4+CD25+Foxp3+ Treg cells in the peripheral blood of mice, with all populations of cells gated according to an FMO control (FIG. 12A). The results showed that the TNFR2 HuGEMM mouse did not express mTNFR2, and the expression profile/level of hTNFR2 on the surface of CD4+ T cells, CD8+ T cells and Treg cells was consistent with that of mTNFR2 on a BALB/c background mouse, indicating that the replacement of mTNFR2 with hTNFR2 was achieved in the TNFR2 HuGEMM mouse (FIGs. 12B-12C).

### Example 14 Inhibition of TNFα-induced proliferation of Treg cells in TNFR2 HuGEMM by an anti-TNFR2 antibody

By measuring the TNFα-induced proliferation of Treg cells in a TNFR2 HuGEMM mouse in the presence of a TNFR2 antibody, TNFR2 signalling pathway and corresponding functions of the TNFR2 HuGEMM mouse genetically engineered were verified to be retained, so that it was ensured that the engineered transgenic mouse could be used to evaluate the *in vivo* efficacy of a candidate anti-TNFR2 antibody. The spleens from 5 TNFR2 HuGEMM mice and 5 wild-type Balb/c mice were extracted aseptically in a biosafety cabinet for grinding. All the grinded cell suspensions were transferred to a 15 ml centrifuge tube for centrifugation (at 320g and 4°C, for 7 minutes). The supernatant was poured out. About 2-3 ml of ACK erythrocyte lysate (Gibco, Cat# A1049201) was added, and the mixture was mixed well, and kept at room temperature for 3-5 minutes until erythrocytes were completely lysed; 6-8 ml of RPMI1640 medium was added to stop the lysis, and the mixture was mixed well by inversion. The well-mixed mixture was subjected to centrifugation (at 200 g and 4°C, for 8 minutes). After the centrifugation, the supernatant was poured out, and the cells were resuspended with EasySep buffer, filtered with a 40 µm filter membrane, and counted. Then Treg cells were sorted according to the instruction of Mouse CD4+CD25+ Regulatory T kit (brand: Stemcell, Cat# 18783A). The sorted Treg cells were stained with CellTrace Violet cell proliferation kit (Thermo, Cat# C34557), with 1 µl of storage solution per 1 × 10⁷ cells used to prepare 1 ml of staining solution. Staining was performed at 37°C for 20 min; the reaction was neutralized with at least 5 times the volume of complete medium, left to stand for 5 min, and centrifuged at 400×g for 5 min. The cells were resuspended and washed once with a medium, and then the washed cells were centrifuged again to obtain the supernatant. The cells were plated at a density of 1 × 10⁵ cells per well of a 96-well plate, resuspended with a medium at a density of 1 × 10⁵ cells per 50 µl, and transferred to a 96-well plate. 50 µl of antibodies to be tested (including three humanized antibodies against TNFR2, an anti-murine TNFR2 surrogate antibody Surrogate antibody clone#75-54.7 (BioXcell, BE0247), and an isotype control antibody anti-Hel-mIgG1) were added to each well, with the final concentration of the antibodies being 10 µg/ml; the mixture was incubated in an incubator for 30 minutes. Then 50 µl of medium containing mouse IL-2 (brand: Sino Biological Inc., Cat# 51061-MNAE, with a final concentration of 300 ill) and mouse TNFα (brand: Sino Biological Inc., Cat# 20180502, with a final concentration of 50 ng/ml) and 50 µl of medium without or with Dynabeads mouse T-activator CD3/CD28 (Gibco, Cat# 11452D, with the ratio of beads to Treg cells of 1 : 20) were added to each well, with the final volume of each well reaching 200 µl. In addition, control wells with Treg cells added with mouse IL-2 only, Treg cells added with mouse TNFα only, and Treg cells added with both mouse IL-2 and human TNFα were set. Two parallel wells were set for each condition; the above-mentioned well plates were mixed evenly, and then incubated in an incubator at 37°C for three days for FACS detection (Thermo Attune NxT). The inhibitory effect of the antibody on TNFα-induced Treg proliferation was determined by the proportion of Treg proliferation. FIG. 13A shows that Treg cells of a TNFR2 HuGEMM mouse express human TNFR2, but no murine TNFR2; Treg cells of a WT Balb/c mouse only express murine TNFR2. FIGs. 13B and 13C show that the proliferation of TNFR2 HuGEMM Treg increases obviously when mIL-2 and mTNFα or hTNFα are added, regardless of the presence of Dynabeads mouse T-activator CD3/CD28 agonist in the system, indicating that the TNFR2 HuGEMM mouse genetically engineered maintains the TNFR2-mediated signals; however, the proliferation of WT balb/c only increases when mIL-2 and mTNFα are added, but hTNFα does not affect the proliferation, indicating that hTNFα does not bind to mTNFR2. In the presence of mIL-2 and mTNFα, humanized antibodies against TNFR2 were added to TNFR2 HuGEMM Treg; compared with the negative control anti-Hel-mIgG1, 001-Hu3-mIgG1 and 219-Hul-mIgG1 could significantly inhibit the proliferation of TNFR2 HuGEMM Treg, with statistical differences (*** p < 0.001), while the anti-murine TNFR2 surrogate antibody TR75-54.7 exhibited no inhibitory effect; in the presence of mIL-2 and mTNFα, anti-TNFR2 antibodies were added to WT Balb/c Treg; compared with the negative control Hamster IgG, the anti-murine TNFR2 surrogate antibody TR75-54.7 could significantly inhibit the proliferation of WT Balb/c Treg, with statistical differences (*** p < 0.001), while 001-Hu3-mIgG1 and 219-Hul-mIgG1 both exhibited no inhibitory effect.

### Example 15 Efficacy evaluation of an anti-TNFR2 antibody in a TNFR2 HuGEMM mouse tumour model

An EGE system developed based on CRISPR/Cas9 by Biocytogen Gene Biotechnology Co., Ltd. was used to replace part of Exon2 and Exon3 of mTNFR2 gene in a mouse CT26 cell line with PuroR cassette, so as to achieve the purpose of gene knockout and destroy the expression of endogenous mTNFR2 in the mouse; then the CDS of a chimeric protein from a mouse intracellular region, transmembrane region and a human extracellular region was inserted to prepare the CT26 cell line only expressing hTNFR2 gene (CT26-hTNFR2 KI). A colon cancer animal model in which hTNFR2 gene was knocked into the CT26-hTNFR2 KI cell line was established by using TNFR2 HuGEMM, and an efficacy experiment of an anti-TNFR2 antibody was carried out.

A CT26-hTNFR2 KI cell was taken out from the cell bank, and recovered with a complete 1640 medium (1640 (Gibco, 61870-036) + 10% FBS (Gibco, 10099-141C) + 1% P/S (Gibco, 15140-122) + 1% NEAA (Gibco, 11140-050) + 1% Sodium Pyruvate (Gibco, 11360-070) + 16 µg/ml puromycin (Gibco, A11138-03)); the recovered cell was placed in a cell culture bottle (with cell type, number of generation, date, name of the person performing the culture, etc. marked on the cell bottle wall) and incubated in a CO₂ incubator (at a temperature of 37°C and a CO₂ concentration of 5%). Cells were passaged upon covering 80% - 90% of the bottom of the culture bottle; after the passage, the cells were continued to be cultured in the CO₂ incubator; the process was repeated until the number of cells met the requirements of an *in vivo* efficacy experiment; subsequently, a cell suspension was prepared, and counted by an automatic cell counter (BECKMAN, Vi-Cell XR); according to the counting results, the cells were resuspended with a PBS solution (HyClone, SH30256.01) to prepare a cell suspension (at a density of 2 × 10⁶ cells/ml). The above cell suspension was inoculated subcutaneously on the right flank of a mouse at 100 µl/mouse. When the tumour volume reached about 100 mm³, mice with moderate individual tumour volume were selected into groups, and the animals were divided into 8 experimental groups according to the tumour volume using an EXCEL random number based randomization method, see Table 9 for grouping. Dosing was started on the same day (marked as Day 0), with the dosing volume of 10 ml/kg; PBS was used as a vehicle; and the dosing was performed by intraperitoneal injection at a frequency of once every 3 days for a total of 4 doses. The tumour volume and body weight were measured three times a week at a fixed time. The tumour volume is calculated according to the following equation: Tumour volume (mm³) = length (mm) × width (mm) × width (mm)/2, and the tumour inhibition rate is calculated according to the following equation: Equation to calculate the tumour inhibition rate: TGI (%) = [1-T/C]×100%, with a statistical method of Two-way ANOVA used to analyse the experimental data. On Day 11 after the dosing, all dosage groups of the test anti-TNFR2 antibody group had statistical differences compared with the PBS control group, with an obvious efficacy. In addition, there was a dose-dependent relationship between different dosage groups of the same antibody. Moreover, the efficacy of the positive drug anti-mPD-1 (BioXcell, BE0146) at 10 mg/kg was comparable to that of the test anti-TNFR2 antibodies 219-Hu1-mIgG2a and 001-Hu3-mIgG2a at a dose of 3 mg/kg. All these results showed that the test humanized antibodies against TNFR2 had a significant *in vivo* tumour growth inhibition efficacy and were dose-dependent (A and B in FIG. 14, and Table 10). Furthermore, during the dosing period, the experimental animals in each group were in good activity and eating state, with a certain degree of increase in body weight and weight change rate, indicating a low *in vivo* safety risk of the test humanized antibodies against TNFR2 (A-D in FIG. 15).

**Table 9. Grouping of TNFR2 HuGEMM mice in an in vivo efficacy experiment**

| **Group** | **Number (mouse/mice)** | **Test sample** | **Inoculation amount of cell** | **Dose (mpk)** |
|---|---|---|---|---|
| G1 | 8 | Vehicle control group (PBS) | 0.2m/mice | 10 ml/kg |
| G2 | 8 | 219-Hu1-mIgG2a | | 3 mg/kg |
| G3 | 8 | | | 10 mg/kg |
| G4 | 8 | | | 30 mg/kg |
| G5 | 8 | 001-Hu3-mIgG2a | | 3 mg/kg |
| G6 | 8 | | | 10 mg/kg |
| G7 | 8 | | | 30 mg/kg |
| G8 | 8 | anti-mPD-1 | | 10 mg/kg |

**Table 10. Dose-effect relationship of in vivo tumour inhibition in TNFR2 HuGEMM mice**

| **Group** | **Test sample** | **Number of animal (mouse/mice)** | **Number of dosing** | **Tumour inhibition rate (%)** | **Dose (mg/kg)** |
|---|---|---|---|---|---|
| G1 | Vehicle control group | 8 | 4 | - | - |
| G2 | 219-Hu1-mIgG2a | 8 | 4 | 25.9 | 3 |
| G3 | | 8 | 4 | 52.9 **** | 10 |
| G4 | | 8 | 4 | 72.5 **** | 30 |
| G5 | 001-Hu3-mIgG2a | 8 | 4 | 30.1 * | 3 |
| G6 | | 8 | 4 | 45.9 *** | 10 |
| G7 | | 8 | 4 | 50.7 *** | 30 |
| G8 | anti-mPD-1 | 8 | 4 | 20.9 | 10 |

| | | | | | |
|---|---|---|---|---|---|
| *: p < 0.05, ***: p < 0.001, ****: p < 0.0001, Two-way ANOVA, vs. G1 PBS control group | | | | | |

### Example 16 Efficacy evaluation of an anti-TNFR2 antibody in a humanized mouse colon cancer tumour model with immune reconstruction of human PBMCs

NOG female mice (purchased from Beijing Vital River Laboratory Animal Technology Co., Ltd.) aged 7-9 weeks were subcutaneously inoculated with 3.5 × 10⁶ HT-29 cells (set as Day 0 of the experiment), and 7 × 10⁶ human peripheral blood mononuclear cells (PBMCs, donor #193 and donor #272, extracted from the screened whole blood of donors by CrownBio (Taicang) Co., Ltd. according to SOP-CP-042) were intraperitoneally injected to the mice on the same day to establish a PBMC humanized colon cancer model. When the tumour volume reached about 95 mm³, mice with moderate individual tumour volume were selected into groups, and the animals were randomized into 5 experimental groups (with G1 as a PBS control group, G2 as a control antibody anti-Hel hIgG1 group, G3 as an anti-TNFR2 antibody 001-Hu3-hIgG1 group, G4 as an anti-TNFR2 antibody 219-Hu1-hIgG1 group, and G5 as a Keytruda group) according to the tumour volume using Study DirectorTM (version No.: 3.1.399.19, supplied by Studylog System, Inc., S. San Francisco, CA, USA), with 5-7 mice in each group for two donors for *in vivo* efficacy study. Dosing was started on the day of grouping (Day 13). The dose was 10 mg/kg (with the same meaning as the measuring unit mpk in the figure, the same below), and the drug was injected intraperitoneally twice a week. The tumour volume and body weight were measured twice a week at a fixed time. The tumour volume (TV) is calculated according to the following equation: V = 1/2×a×b², where a and b represent length and width, respectively. In donor #193, on Day 29 after the tumour inoculation, compared with the control antibody group, the group 219-Hu1 had tumour growth significantly inhibited, with statistical differences (P < 0.0001); there was no statistical difference between other treatment groups and the control antibody group. It shows that in donor #193, the anti-TNFR2 antibody 219-Hu1 has a significant efficacy (A in FIG. 16). In donor #272, on Day 29 after the tumour inoculation, compared with the control antibody group, the groups of 001-Hu3, 219-Hu1, and Keytruda all had tumour growth significantly inhibited, with statistical differences (P < 0.0001). It shows that in donor #272, both Keytruda and anti-TNFR2 antibodies 001-Hu3 and 219-Hu1 exhibit an efficacy (B in FIG. 16).

During the experiment, both the control group and the dosing groups exhibited weight loss and death of mice. According to the results of veterinary autopsy, it was inferred that the death of mice was due to a graft versus host disease (GvHD) reaction caused by injection of PBMCs. At the test dose of 10 mg/kg, the test drugs 001-Hu3 and 219-Hu1 and the positive drug Keytruda produced no obvious drug toxicity as observed in animals, and the treatment was tolerable (C-D in FIG. 16).

48 h after the last dosing, mice in the G2 control antibody group, G3 anti-TNFR2 antibody 001-Hu3 group and G4 anti-TNFR2 antibody 219-Hu1 group were selected for tumour infiltrating lymphocyte (TIL) isolation (Miltenyi, Cat# 130-095-929), so as to perform immunophenotyping by FACS; 123 count eBeads (eBioscience, Cat# 01-1234-42) was added for absolute cell counting, with all populations of cells gated according to an FMO control (A in FIG. 17). The experimental results showed that compared with the control antibody group, the anti-TNFR2 antibody treatment group in donor #193 had the decreased proportion of CD4+ T cells, with no significant difference in the number, and there was no significant difference in the proportion and number of CD4+ T cells in the anti-TNFR2 antibody treatment group in donor #272 (B-C and E-F in FIG. 17). Compared with the control antibody group, the anti-TNFR2 antibody treatment group in donor #193 had the increased proportion of CD8+ T cells, with no significant difference in the number and proportion of CD8+ T cells, but the number of CD8+ T cells in the antibody treatment group 001-Hu3 increased significantly (B-C and E-F in FIG. 17). The results of two donors both showed that the proportion and number of Treg cells (CD4+CD25+Foxp3+ cells) in the anti-TNFR2 antibody treatment group were significantly lower than those in the control antibody group, with significant differences (B-C and E-F in FIG. 17). Moreover, the ratio of total CD8+T cells to Treg cells (CD8+ T/Treg) in the anti-TNFR2 antibody treatment group was also significantly higher than that in the control antibody group, with significant differences (D and G in FIG. 17). The above data show that the anti-TNFR2 antibody treatment can significantly increase the CD8+T/Treg ratio in TILs, thus inhibiting the tumour growth.

### Example 17 Efficacy test of a 219-Hu1-hIgG1 monoclonal antibody in combination with an anti-murine PD-L1 monoclonal antibody in a TNFR2 humanized BALBc mouse model

An animal model of CT26.1C03 colon cancer cells engineered with subcutaneous inoculation of human TNFR2 gene (hTNFR2 KI-CT26) in genetically engineered BALB/c mice (TNFR2 HuGEMM) expressing human TNFR2 was used to evaluate the combined efficacy experiment of the 219-Hu1-hIgG1 antibody and the anti-murine PD-L1 (anti-mPD-L1, BioXcell, BE0101). Mice were subcutaneously inoculated with 5 × 10⁵ CT26.1C03 colon cancer cells on the right flank (set as Day 0 of the experiment). When the tumour volume reached about 100 mm³, mice with moderate individual tumour volume were selected into groups, and the animals were divided into 5 experimental groups (with G1 as a control group, G2 as an anti-mPD-L1 10 mg/kg antibody group, G3 as a 219-Hu1-hIgG1 10 mg/kg antibody group, G4 as a 219-Hu1-hIgG1 30 mg/kg antibody group, and G5 as a combination group of 219-Hu1-hIgG1 10 mg/kg + anti-mPD-L1 10 mg/kg antibodies) according to the tumour volume using an EXCEL random number based randomization method, with 10 mice in each group for *in vivo* efficacy study. Dosing was started on the day of grouping (Day 10). Dosing was performed by intraperitoneal injection twice a week. The tumour volume and body weight were measured three times a week at a fixed time.

On Day 18 after the tumour inoculation (i.e., Day 8 of dosing), compared with the control group (G1), the G2 anti-mPD-L1 10 mg/kg group, the G3 219-Hu1-hIgG1 10 mg/kg group and the G4 219-Hu1-hIgG1 30 mg/kg group had TGI_{TV} values of 35.1%, 28.2% and 63.8%, respectively, suggesting that the tumour volume growth was all significantly inhibited in all three treatment groups (G2 vs. G1, P < 0.0001; G3 vs. G1, P = 0.0002; G4 vs. G1, P < 0.0001); the 219-Hu1-hIgG1 10 mg/kg group and the anti-mPD-L1 10 mg/kg group had comparable TGI_{TV} values; however, the 219-Hu1-hIgG1 30 mg/kg group had a TGI_{TV} value significantly higher than that in the 219-Hu1-hIgG1 10 mg/kg group and the anti-mPD-L1 10 mg/kg group (G2 vs. G4, P = 0.0002; G3 vs. G4, P < 0.0001). The above results showed that the 219-Hu1-hIgG1 group had a dose-dependent effect within the dose range of 10 - 30 mg/kg, with tumour volume decreased as the increase of dose. See Table 11 and FIG. 18 for details.

On Day 18 after the tumour inoculation, compared with the control group (G1), the combined dosing group of G5 219-Hu1-hIgG1 10 mg/kg + anti-mPD-L1 10 mg/kg also had the tumour volume growth significantly inhibited (TGI_{TV} = 66.5, vs. G1, P < 0.0001); in addition, the combined dosing group of G5 219-Hu1-hIgG1 10 mg/kg + anti-mPD-L1 10 mg/kg also had a mean tumour volume significantly smaller than that in the single-dose groups of G3 219-Hu1-hIgG1 10 mg/kg and G2 anti-mPD-L1 10 mg/kg (TGI_{TV} G5 vs. G3 vs. G2 = 66.5% vs. 28.2% vs. 35.1%; G5 vs. G2, P < 0.0001; G5 vs. G3, P < 0.0001), indicating that the combined dosing of the 219-Hu1-hIgG1 antibody and the anti-mPD-L1 antibody inhibited the tumour growth more potently compared with two single-dose groups, with statistical differences (****, p < 0.0001, single-dose group vs. combined dosing group). See Table 11 and FIG. 18 for details.

**Table 11. Evaluation of anti-tumour effect of 219-Hu1-hIgG1 and/or anti-mPD-L1 on a CT26.1C03 allograft tumour model (calculated based on tumour volume on Day 18 after inoculation)**

| **Group** | **Number of animal** | **Tumour volume (mm³)^{a} (Day 10)** | **Tumour volume (mm³)^{a} (Day 18)** | **RTV^{b} (Day 18/Day 10)** | **T/C^{b} (%)** | **TGI_{TV}^{c} (%)** | **P-value (vs. G1 group)^{d}** | **P-value (vs. G5 group)^{e}** | **P-value (vs. G4 group)^{f}** |
|---|---|---|---|---|---|---|---|---|---|
| G1 | 10 | 92.79±16. 02 | 1783.49±383. 84 | 19.22 | -- | -- | -- | <0.0001 | <0.0001 |
| G2 | 10 | 93.84±15. 48 | 1191.17±797. 12 | 12.69 | 66.02 | 35.1 | <0.0001 | <0.0001 | 0.0002 |
| G3 | 10 | 92.97±18. 61 | 1307.01±484. 68 | 14.06 | 73.15 | 28.2 | 0.0002 | <0.0001 | <0.0001 |
| G4 | 10 | 93.36±17. 82 | 705.84±443.7 4 | 7.56 | 39.33 | 63.8 | <0.0001 | 0.7155 | -- |
| G5 | 10 | 93.23±18. 12 | 659.61±377.5 7 | 7.08 | 36.84 | 66.5 | <0.0001 | **--** | 0.7155 |

| | | | | | | | | | |
|---|---|---|---|---|---|---|---|---|---|
| Notes: a. Mean ± SD. b. The relative tumour growth rate T/C % = T_{RTV}/C_{RTV} × 100 % (T_{RTV}: mean RTV in the treatment groups; C_{RTV}: mean RTV in the negative control group). According to the results of tumour measurement, the relative tumour volume (RTV) is calculated with the calculation equation of RTV = Vₜ/V₀, where V₀ is the tumour volume measured at the time of grouping and dosing (i.e., PO-D0) and Vₜ is the tumour volume measured at a certain time. c. TGI_{TV} (%) = [1-(Ti-T0)/(Vi-V0)]×100% (Ti: mean tumour volume of treatment groups on Day i of dosing; T0: mean tumour volume of treatment groups on Day 0 of dosing; Vi: mean tumour volume of a solvent control group on Day i of dosing; V0: mean tumour volume of a solvent control group on Day 0 of dosing). d. Data are analysed using Two-way ANOVA, in which when compared with G1 Veh, p < 0.05 indicates a significant difference; ***: p < 0.001, ****: p < 0.0001. e. Data are analysed using Two-way ANOVA, in which when compared with the combined dosing group of G5, p < 0.05 indicates a significant difference; ****: p < 0.0001. f. Data are analysed using Two-way ANOVA, in which when compared with the G4 219-Hu1-hIgG1 30 mpk group, p < 0.05 indicates a significant difference; ***: p < 0.001, ****: p < 0.0001. | | | | | | | | | |

During the dosing period, the experimental animals in each group were in good activity and eating state, with a certain degree of increase in body weight. During the observation in the experimental period, no deterioration of animal mental state, activity and body surface observation was found in each group, and all the animals showed no drug toxicity and showed good safety results, indicating that the safety of the 219-Hu1-hIgG1 antibody and the combined dosing group of 219-Hu1-hIgG1 + anti-mPD-L1 antibodies was high. See FIGs. 19-20 and Table 12 for details.

**Table 12. Effect of 219-Hu1-hIgG1 and/or anti-mPD-L1 on body weight of mice in a CT26.1C03 allograft tumour model**

| **Group** | **Number of animal at the start of experiment/at the end of experiment** | **Mean body weight (g)^{a} (Day 0 after grouping)** | **Mean body weight (g)^{a} (Day 8 after grouping)** | **Mean weight change rate (%, Day 8 after grouping)** |
|---|---|---|---|---|
| G1 | 10/10 | 20.7±1.0 | 23.7±1.3 | 14.6 |
| G2 | 10/10 | 20.2±1.5 | 22.7±1.8 | 12.3 |
| G3 | 10/10 | 20±1.4 | 22.5±1.4 | 13.2 |
| G4 | 10/10 | 20.5±1.5 | 22.1±1.5 | 8.3 |
| G5 | 10/10 | 19.6±0.8 | 21.1±1.8 | 7.8 |

| | | | | |
|---|---|---|---|---|
| Notes: mean ± SD. | | | | |

On Day 18 after the tumour inoculation, the animals in each group were euthanized, and the tumours were isolated and weighed. Compared with the control group (G1), the G2 anti-mPD-L1 10 mg/kg group had the tumour weight significantly reduced (TGI_{TW} = 37.3%, P-value: 0.0087); the G3 219-Hu1-hIgG1 10 mg/kg group had a tumour weight lower than that of the control group, without a statistical difference (TGI_{TW} = 25.9%, P-value: 0.0830); the G4 219-Hu1-hIgG1 30 mg/kg group also had a tumour weight significantly lower than that of the control group (TGI_{TW} = 47.9%, P-value: 0.0013); the combined dosing group of G5 219-Hu1-hIgG1 10 mg/kg + anti-mPD-L1 10 mg/kg also had a tumour weight significantly lower than that of the control group (TGI_{TW} = 43.0%, P-value: 0.0042). The tumour weight of 219-Hu1-hIgG1 at two doses had a dose-dependent effect, with tumour weight decreased as the increase of dose. The tumour weight of the combined dosing group was lower than that of each single-dose group (G2 and G3), without a statistical difference (FIG. 21, and Table 13).

**Table 13. Evaluation of experimental end-point anti-tumour effect of 219-Hu1-hIgG1 and/or anti-mPD-L1 on a CT26.1C03 allograft tumour model (calculated based on tumour weight on Day 18 after inoculation)**

| **Group** | **Number of animal** | **Tumour weight (g) ^{a} (Day 18)** | **TGI_{Tw}^{b} (%)** | **P-value^{c}** |
|---|---|---|---|---|
| G1 | 10 | 2.507±0.611 | -- | -- |
| G2 | 10 | 1.571±0.797 | 37.3 | 0.0087 |
| G3 | 10 | 1.857±0.784 | 25.9 | 0.0830 |
| G4 | 10 | 1.306±0.902 | 47.9 | 0.0013 |
| G5 | 10 | 1.429±0.872 | 43.0 | 0.0042 |

| | | | | |
|---|---|---|---|---|
| Notes: a. Mean ± SD. b. TGI_{Tw} (%) = [1-Ti/Vi]×100% (Ti: mean tumour weight of treatment groups on Day i of dosing; Vi: mean tumour weight of a solvent control group on Day i of dosing). c. Data are analysed using a One-way ANOVA method, in which when compared with G1 Veh, p < 0.05 indicates a significant difference; **: p < 0.01. | | | | |

## Claims

1. A humanized antibody that specifically binds to TNFR2, or an antigen-binding fragment thereof, **characterized in that** the antibody or antigen-binding fragment thereof comprises a heavy chain variable region (VH) and a light chain variable region (VL);
(1) the heavy chain variable region comprises:
a. a VH sequence selected from any of SEQ ID NOs: 19-27,
b. a VH sequence selected from a sequence having at least 80%, 85%, 90%, 91%, 92%, 93%, 94%, 95%, 96%, 97%, 98%, 99% or 100% sequence identity to any of SEQ ID NOs: 19-27, or,
c. a VH sequence selected from a sequence having 1, 2, 3, 4, 5, 6, 7, 8, 9, 10 or more amino acid insertions, deletions and/or substitutions relative to any of SEQ ID NOs: 19-27;
(2) the light chain variable region comprises:
a. a VL sequence selected from any of SEQ ID NOs: 28-37,
b. a VL sequence selected from a sequence having at least 80%, 85%, 90%, 91%, 92%, 93%, 94%, 95%, 96%, 97%, 98%, 99% or 100% sequence identity to any of SEQ ID NOs: 28-37, or,
c. a VL sequence selected from a sequence having 1, 2, 3, 4, 5, 6, 7, 8, 9, 10 or more amino acid insertions, deletions and/or substitutions relative to any of SEQ ID NOs: 28-37;
optionally, the amino acid insertions, deletions and/or substitutions occur in an FR region of the heavy chain variable region or/and the light chain variable region;
optionally, the substitutions are substitutions of conservative amino acids.

2. The antibody or antigen-binding fragment thereof according to claim 1, **characterized in that** the heavy chain variable region comprises a VH sequence with a CDR identical to and an FR having at least 85%, 90%, 91%, 92%, 93%, 94%, 95%, 96%, 97%, 98%, 99% or 100% sequence identity to those of any of SEQ ID NOs: 19-27; and/or the light chain variable region comprises a VL sequence with a CDR identical to and an FR having at least 85%, 90%, 91%, 92%, 93%, 94%, 95%, 96%, 97%, 98%, 99% or 100% sequence identity to those of any of SEQ ID NOs: 28-37.

3. The antibody or antigen-binding fragment thereof according to claim 1, **characterized in that** the heavy chain variable region and the light chain variable region are selected from the group consisting of:
(1) VH having a sequence as shown in SEQ ID NO: 19 and VL having a sequence as shown in SEQ ID NO: 28;
(2) VH having a sequence as shown in SEQ ID NO: 20 and VL having a sequence as shown in SEQ ID NO: 29;
(3) VH having a sequence as shown in SEQ ID NO: 19 and VL having a sequence as shown in SEQ ID NO: 30;
(4) VH having a sequence as shown in SEQ ID NO: 20 and VL having a sequence as shown in SEQ ID NO: 31;
(5) VH having a sequence as shown in SEQ ID NO: 19 and VL having a sequence as shown in SEQ ID NO: 31;
(6) VH having a sequence as shown in SEQ ID NO: 21 and VL having a sequence as shown in SEQ ID NO: 32;
(7) VH having a sequence as shown in SEQ ID NO: 22 and VL having a sequence as shown in SEQ ID NO: 32;
(8) VH having a sequence as shown in SEQ ID NO: 23 and VL having a sequence as shown in SEQ ID NO: 32;
(9) VH having a sequence as shown in SEQ ID NO: 24 and VL having a sequence as shown in SEQ ID NO: 32;
(10) VH having a sequence as shown in SEQ ID NO: 25 and VL having a sequence as shown in SEQ ID NO: 32;
(11) VH having a sequence as shown in SEQ ID NO: 26 and VL having a sequence as shown in SEQ ID NO: 33;
(12) VH having a sequence as shown in SEQ ID NO: 27 and VL having a sequence as shown in SEQ ID NO: 34;
(13) VH having a sequence as shown in SEQ ID NO: 27 and VL having a sequence as shown in SEQ ID NO: 35;
(14) VH having a sequence as shown in SEQ ID NO: 27 and VL having a sequence as shown in SEQ ID NO: 33;
(15) VH having a sequence as shown in SEQ ID NO: 27 and VL having a sequence as shown in SEQ ID NO: 36;
(16) VH having a sequence as shown in SEQ ID NO: 26 and VL having a sequence as shown in SEQ ID NO: 34;
(17) VH having a sequence as shown in SEQ ID NO: 27 and VL having a sequence as shown in SEQ ID NO: 37;
(18) a combination of VH and VL having at least 80%, 85%, 90%, 91%, 92%, 93%, 94%, 95%, 96%, 97%, 98%, 99% or 100% sequence identity to a combination of sequences of any of the above (1) - (17);
(19) a combination of VH and VL having 1, 2, 3, 4, 5, 6, 7, 8, 9, 10 or more amino acid insertions, deletions and/or substitutions relative to a combination of sequences of any of the above (1) - (17); or,
(20) a combination of VH and VL with a CDR identical to and an FR having at least 85%, 90%, 91%, 92%, 93%, 94%, 95%, 96%, 97%, 98%, 99% or 100% sequence identity to those of a combination of sequences of any of the above (1) - (17);
optionally, the amino acid insertions, deletions and/or substitutions occur in the FR region of the heavy chain variable region or/and the light chain variable region;
optionally, the substitutions are substitutions of conservative amino acids.

4. The antibody or antigen-binding fragment thereof according to any of claims 1-3, **characterized in that** the antibody or antigen-binding fragment thereof comprises:
CDR1-VH selected from the group consisting of SEQ ID NO: 1, 7, and 13;
CDR2-VH selected from the group consisting of SEQ ID NO: 2, 8, and 14;
CDR3-VH selected from the group consisting of SEQ ID NO: 3, 9, and 15;
CDR1-VL selected from the group consisting of SEQ ID NO: 4, 10, and 16;
CDR2-VL selected from the group consisting of SEQ ID NO: 5, 11, and 17; and
CDR3-VL selected from the group consisting of SEQ ID NO: 6, 12, and 18.

5. The antibody or antigen-binding fragment thereof according to any of claims 1-4, **characterized in that** the antibody or antigen-binding fragment thereof binds to human TNFR2 with a dissociation constant (KD) no more than 7 nM, and to cynomolgus monkey TNFR2 with a dissociation constant (KD) no more than 5 nM.

6. The antibody or antigen-binding fragment thereof according to any of claims 1-5, **characterized in that** the antibody or antigen-binding fragment thereof comprises or does not comprise a heavy chain constant region and/or a light chain constant region;
preferably, the heavy chain constant region comprises a full-length heavy chain constant region or a fragment thereof, and the fragment is selected from the group consisting of a CH1 domain, an Fc domain and a CH3 domain;
preferably, the heavy chain constant region and/or the light chain constant region are/is a human heavy chain constant region and/or a human light chain constant region;
preferably, the heavy chain constant region is selected from an IgG heavy chain constant region, such as an IgG1 heavy chain constant region, an IgG2 heavy chain constant region, an IgG3 heavy chain constant region or an IgG4 heavy chain constant region;
preferably, the heavy chain constant region is selected from the group consisting of a human IgG1 heavy chain constant region, a human IgG2 heavy chain constant region, a human IgG3 heavy chain constant region and a human IgG4 heavy chain constant region.

7. The antibody or antigen-binding fragment thereof according to any of claims 1-6, **characterized in that** the antibody or antigen-binding fragment thereof is selected from the group consisting of a monoclonal antibody, a polyclonal antibody, a natural antibody, an engineered antibody, a monospecific antibody, a multispecific antibody (e.g., a bispecific antibody), a monovalent antibody, a multivalent antibody, a full-length antibody, an antibody fragment, Fab, Fab', F(ab')₂, Fd, Fv, scFv, and a diabody.

8. The antibody or antigen-binding fragment thereof according to any of claims 1-7, **characterized in that** the antibody or antigen-binding fragment thereof is coupled with another molecule via or not via a linker; preferably, the another molecule is selected from a therapeutic agent or a tracer; preferably, the therapeutic agent is selected from the group consisting of a radioisotope, a chemotherapeutic drug and an immunomodulator, and the tracer is selected from the group consisting of a radiological contrast agent, a paramagnetic ion, a metal, a fluorescent label, a chemiluminescence label, an ultrasonic contrast agent and a photosensitizer.

9. The antibody or antigen-binding fragment thereof according to any of claims 1-8, **characterized in that** the antibody or antigen-binding fragment thereof:
(1) specifically binds to a cell expressing TNFR2 on the cell surface;
(2) specifically binds to a Treg cell;
(3) inhibits the binding of TNFα to a TNFR2 protein;
(4) inhibits the binding of TNFα to TNFR2 expressed on the cell surface;
(5) inhibits the Treg proliferation and/or Treg function mediated by TNFα;
(6) inhibits the degradation of IκBα mediated by TNFα;
(7) inhibits the inhibition of Tcon cell proliferation by Treg;
(8) mediates the ADCC function against a TNFR2-expressing cell;
(9) increases the ratio of CD8+ T/Treg cells in tumour infiltrating lymphocytes, TILs; or/and,
(10) inhibits the tumour growth.

10. A multispecific antigen-binding molecule, **characterized in that** the multispecific antigen-binding molecule comprises at least a first antigen-binding module and a second antigen-binding module, wherein the first antigen-binding module comprises the antibody or antigen-binding fragment thereof according to any of claims 1-9, and the second antigen-binding module specifically binds to other antigens than TNFR2 or binds to a different TNFR2 antigenic epitope from the first antigen-binding module;
preferably, the other antigens are selected from the group consisting of CD3, CD4, CD5, CD8, CD14, CD15, CD16, CD16A, CD19, CD20, CD21, CD22, CD23, CD25, CD33, CD37, CD38, CD40, CD40L, CD46, CD52, CD54, CD70, CD74, CD80, CD86, CD126, CD138, B7, PD-1, PD-L1, PD-2, CTLA4, PRVIG, TIGHT, HAS, CLDN18.2, MSLN, MUC, Ia, HLA-DR, tenascin, EGFR, VEGF, P1GF, ED-B fibronectin, an oncogene product, IL-2, IL-6, IL-15, IL-21, TRAIL-R1 and TRAII,-R2;
preferably, the multispecific antibody is selected from the group consisting of a bispecific antibody, a trispecific antibody and a tetraspecific antibody.

11. A chimeric antigen receptor (CAR), **characterized in that** the chimeric antigen receptor comprises an extracellular antigen-binding domain, a transmembrane domain and an intracellular signalling domain; the extracellular antigen-binding domain comprises the humanized antibody against TNFR2 or antigen-binding fragment thereof according to any of claims 1-9 or comprises the multispecific antigen-binding molecule according to claim 10.

12. An immune effector cell, **characterized in that** the immune effector cell comprises the chimeric antigen receptor according to claim 11 or a nucleic acid fragment encoding the chimeric antigen receptor according to claim 11;
preferably, the immune effector cell is selected from the group consisting of a T cell, a natural killer (NK) cell, a natural killer T (NKT) cell, a monocyte, a macrophage, a dendritic cell and a mast cell; the T cell is selected from the group consisting of an inflammatory T cell, a cytotoxic T cell, a regulatory T cell (Treg) and a helper T cell;
preferably, the immune effector cell is an allogeneic immune effector cell or an autologous immune cell.

13. An isolated nucleic acid fragment, **characterized in that** the nucleic acid fragment encodes the antibody or antigen-binding fragment thereof according to any of claims 1-9, the multispecific antigen-binding molecule according to claim 10 or the chimeric antigen receptor according to claim 11.

14. A vector, **characterized in that** the vector comprises the nucleic acid fragment according to claim 13.

15. A host cell, **characterized in that** the host cell comprises the vector according to claim 14; preferably, the cell is a prokaryotic cell or a eukaryotic cell, such as a bacterial (*Escherichia coli*) cell, a fungal (yeast) cell, an insect cell or a mammalian cell (a CHO cell line or a 293T cell line); preferably, the cell lacks a fucosyltransferase, and more preferably, the fucosyltransferase is FUT8.

16. A method for preparing the antibody or antigen-binding fragment thereof according to any of claims 1-9 or the multispecific antigen-binding molecule according to claim 10, **characterized in that** the method comprises culturing the host cell according to claim 15, and isolating the antibody or antigen-binding fragment thereof or the multispecific antigen-binding molecule expressed by the host cell.

17. A method for preparing the immune effector cell according to claim 12, **characterized in that** the method comprises introducing a nucleic acid fragment encoding the chimeric antigen receptor (CAR) according to claim 11 into the immune effector cell, and optionally, the method further comprises enabling the immune effector cell to express the CAR according to claim 11.

18. A pharmaceutical composition, **characterized in that** the composition comprises the antibody or antigen-binding fragment thereof according to any of claims 1-9, the multispecific antigen-binding molecule according to claim 10, the chimeric antigen receptor according to claim 11, the immune effector cell according to claim 12, the nucleic acid fragment according to claim 13, the vector according to claim 14 or the cell according to claim 15, and a product prepared from the method according to claim 16 or 17;
preferably, the composition further comprises a pharmaceutically acceptable carrier, a diluent or an auxiliary agent;
preferably, the composition further comprises an additional anti-tumour agent, an immunotherapeutic agent or an immunosuppressant;
preferably, the additional anti-tumour agent is selected from the group consisting of a PD-1 antibody, a PD-L1 antibody and a CTLA-4 antibody.

19. Use of the antibody or antigen-binding fragment thereof according to any of claims 1-9, the multispecific antigen-binding molecule according to claim 10, the chimeric antigen receptor according to claim 11, the immune effector cell according to claim 12, the nucleic acid fragment according to claim 13, the vector according to claim 14, the cell according to claim 15, a product prepared from the method according to claim 16 or 17, and/or the pharmaceutical composition according to claim 18 in the preparation of a drug for treating and/or preventing a disease related to immune abnormalities;
preferably, the disease related to immune abnormalities is related to Treg cells and/or MDSC functions;
preferably, the disease is cancer or an autoimmune disease;
preferably, the cancer is selected from the group consisting of ovarian cancer, advanced epidermal T-cell lymphoma, stage III/IV metastatic colorectal cancer, triple-negative breast cancer, pancreatic cancer, non-small cell lung cancer, and advanced solid tumours resistant to CTLA-4 and PD-1 therapies, e.g., metastatic melanoma;
preferably, the autoimmune disease is selected from the group consisting of rheumatoid arthritis, multiple sclerosis, systemic sclerosis, neuromyelitis optica spectrum disorder, systemic lupus erythematosus, myasthenia gravis and IgG4-related diseases.

20. A method for treating and/or preventing a disease related to immune abnormalities, **characterized in that** the method comprises administering to a subject in need thereof an effective amount of the antibody or antigen-binding fragment thereof according to any of claims 1-9, the multispecific antigen-binding molecule according to claim 10, the chimeric antigen receptor according to claim 11, the immune effector cell according to claim 12, the nucleic acid fragment according to claim 13, the vector according to claim 14, the cell according to claim 15, a product prepared from the method according to claim 16 or 17, and/or the pharmaceutical composition according to claim 18;
preferably, the disease related to immune abnormalities is related to Treg cells and/or MDSC functions;
preferably, the disease is cancer or an autoimmune disease;
preferably, the cancer is selected from the group consisting of ovarian cancer, advanced epidermal T-cell lymphoma, stage III/IV metastatic colorectal cancer, triple-negative breast cancer, pancreatic cancer, non-small cell lung cancer, and advanced solid tumours resistant to CTLA-4 and PD-1 therapies, e.g., metastatic melanoma;
preferably, the autoimmune diseaseis selected from the group consisting of rheumatoid arthritis, multiple sclerosis, systemic sclerosis, neuromyelitis optica spectrum disorder, systemic lupus erythematosus, myasthenia gravis and IgG4-related diseases.

21. The use according to claim 19 or the method according to claim 20, further comprising administering to a subject in need thereof an additional anti-tumour therapeutic agent, such as a chemotherapeutic agent, a targeted therapeutic agent and an immunotherapeutic agent, including a PD-1/PD-L1 therapeutic agent such as an anti-PD-1/PD-L1 antibody, and an anti-CTLA-4 therapeutic agent such as an anti-CTLA-4 antibody;
preferably, the additional anti-tumour therapeutic agent is selected from a PD-1/PD-L1 therapeutic agent;
preferably, the PD-1/PD-L1 therapeutic agent is selected from a PD-1 antibody or a PD-L1 antibody.

22. The antibody or antigen-binding fragment thereof according to any of claims 1-9, the multispecific antigen-binding molecule according to claim 10, the chimeric antigen receptor according to claim 11, the immune effector cell according to claim 12, the nucleic acid fragment according to claim 13, the vector according to claim 14, the cell according to claim 15, a product prepared from the method according to claim 16 or 17, and/or the pharmaceutical composition according to claim 18, for use in treating and/or preventing a disease related to immune abnormalities;
preferably, the disease related to immune abnormalities is related to Treg cells and/or MDSC functions;
preferably, the disease related to immune abnormalities is cancer or an autoimmune disease;
preferably, the cancer is selected from the group consisting of ovarian cancer, advanced epidermal T-cell lymphoma, stage III/IV metastatic colorectal cancer, triple-negative breast cancer, pancreatic cancer, non-small cell lung cancer, and advanced solid tumours resistant to CTLA-4 and PD-1 therapies, e.g., metastatic melanoma;
preferably, the autoimmune disease is selected from the group consisting of rheumatoid arthritis, multiple sclerosis, systemic sclerosis, neuromyelitis optica spectrum disorder, systemic lupus erythematosus, myasthenia gravis and IgG4-related diseases;
preferably, the use further comprises administering to a subject in need thereof an additional anti-tumour therapeutic agent, such as a chemotherapeutic agent, a targeted therapeutic agent and an immunotherapeutic agent, including a PD-1/PD-L1 therapeutic agent such as an anti-PD-1/PD-L1 antibody, and an anti-CTLA-4 therapeutic agent such as an anti-CTLA-4 antibody is further comprised therein.

23. A method for detecting TNFR2 in a sample *in vitro,* comprising the step of contacting a sample suspected of containing TNFR2 with the antibody or antigen-binding fragment thereof according to any of claims 1 to 9.
